# EUROPÄISCHE PATENTSCHRIFT

(11) **EP 1 077 994 B1**
(45) Veröffentlichungstag und Bekanntmachung des Hinweises auf die Patenterteilung: **20.01.2010**
(21) Anmeldenummer: 99923530.2
(22) Anmeldetag: 05.05.1999
(51) Int. Cl.: C07K 5/02, C07D 233/32, A61K 31/415, A61K 38/05

(54) **IMIDAZOLIDINDERIVATE, IHRE HERSTELLUNG, IHRE VERWENDUNG UND SIE ENTHALTENDE PHARMAZEUTISCHE PRÄPARATE**
IMIDAZOLIDINE DERIVATIVES, THE PRODUCTION THEREOF, THEIR USE AND PHARMACEUTICAL PREPARATIONS CONTAINING THE SAME
DERIVES D'IMIDAZOLIDINE, LEUR PRODUCTION, LEUR UTILISATION ET PREPARATION PHARMACEUTIQUE LES CONTENANT

(30) Priorität: 14.05.1998 DE 19821483
(43) Veröffentlichungstag der Anmeldung: 28.02.2001
(73) Patentinhaber: Sanofi-Aventis Deutschland GmbH, 65929 Frankfurt am Main (DE)
(72) Erfinder: NEISES, Bernhard, D-77652 Offenburg (DE); WEHNER, Volkmar, D-97657 Sandberg (DE); STILZ, Hans, Ulrich, D-65929 Frankfurt am Main (DE)
(86) Internationale Anmeldenummer: PCT/EP1999/003072
(87) Internationale Veröffentlichungsnummer: WO 1999/060015

(56) Entgegenhaltungen:
- EP-A- 0 903 353
- EP-A- 0 905 139
- WO-A-95/14008
- DE-A- 4 126 277
- DE-A- 19 647 380
- DE-A- 19 647 381
- DE-A- 19 647 382
- DE-A- 19 751 251
- CHEMICAL ABSTRACTS, vol. 129, no. 18, 2. November 1998 (1998-11-02) Columbus, Ohio, US; abstract no. 225489, H U STILZ ET AL.: "From a peptide lead to an orally active peptidomimetic fibrinogen receptor antagonist " XP002114309 & LETTERS IN PEPTIDE SCIENCE, Bd. 5, Nr. 2-3, Mai 1998 (1998-05), Seiten 215-221, ESCOM SCIENCE PUBLISHERS, NL ISSN: 0929-5666

## Beschreibung

Die vorliegende Erfindung betrifft lmidazolidinderivate der Formel 1, 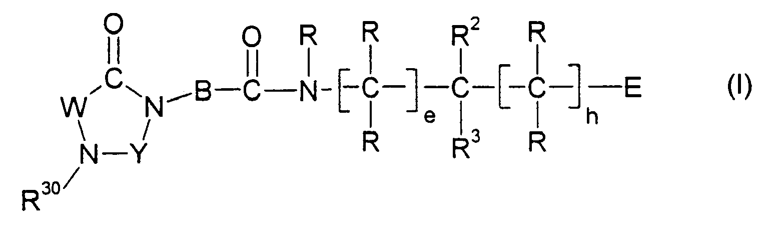 in der B, E, W, Y, R, R², R³, R³⁰, e und h die unten angegebenen Bedeutungen haben. Die Verbindungen der Formel I sind wertvolle Arzneimittelwirkstoffe, die sich zum Beispiel zur Therapie und Prophylaxe von Entzündungserkrankungen, beispielsweise der rheumatoiden Arthritis, oder von allergischen Erkrankungen eignen. Die Verbindungen der Formel I sind Inhibitoren der Adhäsion und Migration von Leukozyten und/oder Antagonisten des zur Gruppe der Integrine gehörenden Adhäsionsrezeptors VLA-4. Sie eignen sich generell zur Therapie oder Prophylaxe von Krankheiten, die durch ein unerwünschtes Ausmaß an Leukozytenadhäsion und/oder Leukozytenmigration verursacht werden oder damit verbunden sind oder bei denen Zell-Zell- oder Zell-Matrix-Interaktionen eine Rolle spielen, die auf Wechselwirkungen von VLA-4-Rezeptoren mit ihren Liganden beruhen. Die Erfindung betrifft weiterhin Verfahren zur Herstellung der Verbindungen der Formel I, ihre Verwendung, insbesondere als Arzneimittelwirkstoffe, und pharmazeutische Präparate, die Verbindungen der Formel I enthalten.

Die Integrine sind eine Gruppe von Adhäsionsrezeptoren, die bei Zell-Zellbindenden und Zell-Extrazelluläre Matrix-bindenden Prozessen eine wesentliche Rolle spielen. Sie weisen eine αβ-heterodimere Struktur auf und zeigen eine weite zelluläre Verbreitung und ein hohes Maß an evolutiver Konservierung. Zu den Integrinen gehört zum Beispiel der Fibrinogen-Rezeptor auf Thrombozyten, der vor allem mit der RGD-Sequenz des Fibrinogens interagiert, oder der Vitronectin-Rezeptor auf Osteoclasten, der vor allem mit der RGD-Sequenz des Vitronectins oder des Osteopontins interagiert. Man teilt die Integrine in drei Großgruppen ein, die β2-Unterfamilie mit den Vertretern LFA-1, Mac-1 und p150/95, die insbesondere für Zell-Zell-Interaktionen des Immunsystems verantwortlich sind, und die Unterfamilien β1 und β3, deren Vertreter hauptsächlich die Zellanheftung an Komponenten der extrazellulären Matrix vermitteln (Ruoslahti, Annu. Rev. Biochem. 1988, 57, 375). Die Integrine der β1-Unterfamilie, auch VLA-Proteine (very late (activation) antigen) genannt, umfassen mindestens sechs Rezeptoren, die spezifisch mit Fibronektin, Kollagen und/oder Laminin als Liganden interagieren. Innerhalb der VLA-Familie ist das Integrin VLA-4 (α4β1) insofern untypisch, als es hauptsächlich auf lymphoide und myeloide Zellen begrenzt ist und bei diesen verantwortlich ist für Zell-Zell-Interaktionen mit einer Vielzahl von anderen Zellen. VLA-4 vermittelt zum Beispiel die Interaktion von T- und B-Lymphozyten mit dem Heparin II-Bindungsfragment von humanem Plasmafibronektin (FN). Die Bindung von VLA-4 mit dem Heparin II-Bindungsfragment des Plasmafibronektins beruht vor allem auf einer Interaktion mit einer LDVP-Sequenz. Im Unterschied zum Fibrinogen- oder Vitronectin-Rezeptor ist VLA-4 kein typisches RGD-bindendes Integrin (Kilger und Holzmann, J. Mol. Meth. 1995, 73, 347).

Die im Blut zirkulierenden Leukozyten zeigen normalerweise nur eine geringe Affinität zu den vaskulären endothelialen Zellen, die die Blutgefäße auskleiden. Zytokine, die von entzündetem Gewebe abgegeben werden, bewirken die Aktivierung von Endothelzellen und damit die Expression einer Vielzahl von Zelloberflächenantigenen. Diese umfassen zum Beispiel die Adhäsionsmoleküle ELAM-1 (endothelial cell adhesion molecule-1; auch als E-Selektin bezeichnet), das unter anderem Neutrophile bindet, ICAM-1 (intercellular adhesion molecule-1), das mit LFA-1 (leucocyte function-associated antigen 1) auf Leukozyten interagiert, und VCAM-1 (vascular cell adhesion molecule-1), das verschiedene Leukozyten, unter anderem Lymphozyten, bindet (Osborn et al., Cell 1989, 59, 1203). VCAM-1 ist, wie ICAM-1, ein Mitglied der Immunglobulin-Gen-Überfamilie. Identifiziert wurde VCAM-1 (zuerst bekannt als INCAM-110) als ein Adhäsionsmolekül, das auf endothelialen Zellen durch Entzündungs-Zytokine wie TNF und IL-1 und Lipopolysaccharide (LPS) induziert wird. Elices et al. (Cell 1990, 60, 577) zeigten, daß VLA-4 und VCAM-1 ein Rezeptor-Ligand-Paar bilden, das die Anheftung von Lymphozyten an aktiviertes Endothel vermittelt. Die Bindung von VCAM-1 an VLA-4 erfolgt dabei nicht durch eine Interaktion des VLA-4 mit einer RGD-Sequenz, eine solche ist im VCAM-1-nicht enthalten (Bergelson et al., Current Biology 1995, 5, 615). VLA-4 tritt aber auch auf anderen Leukozyten auf, und über den VCAM-1/VLA-4-Adhäsionsmechanismus wird auch die Anheftung von anderen Leukozyten als Lymphozyten vermittelt. VLA-4 repräsentiert somit ein einzelnes Beispiel eines β1-Integrin-Rezeptors, der über die Liganden VCAM-1 bzw. Fibronektin sowohl bei Zell-Zell-Interaktionen als auch bei Zell-Extrazellulärer Matrix-Interaktionen eine wesentliche Rolle spielt.

Die Zytokin-induzierten Adhäsionsmoleküle spielen eine wichtige Rolle bei der Rekrutierung von Leukozyten in extravaskuläre Gewebebereiche. Leukozyten werden in entzündliche Gewebebereiche durch Zelladhäsionsmoleküle rekrutiert, die auf der Oberfläche von endothelialen Zellen exprimiert werden und als Liganden für Leukozyten-Zelloberflächen-Proteine oder -Proteinkomplexe (Rezeptoren) dienen (die Begriffe Ligand und Rezeptor können auch vice versa verwendet werden). Leukozyten aus dem Blut müssen zunächst an endotheliale Zellen anheften, bevor sie in das Synovium auswandern können. Da VCAM-1 an Zellen bindet, die das Integrin VLA-4 (α4β1) tragen, wie Eosinophile, T- und B-Lymphozyten, Monozyten oder auch Neutrophile, kommt ihm und dem VCAM-1/ VLA-4-Mechanismus die Funktion zu, derartige Zellen aus dem Blutstrom in Infektionsgebiete und Entzündungsherde zu rekrutieren (Elices et al., Cell 1990, 60, 577; Osborn, Cell 1990, 62, 3; Issekutz et al., J. Exp. Med. 1996, 183, 2175).

Der VCAM-1/VLA-4-Adhäsionsmechanismus wurde mit einer Reihe von physiologischen und pathologischen Prozessen in Verbindung gebracht. VCAM-1 wird außer von Zytokin-induziertem Endothel unter anderem noch von den folgenden Zellen exprimiert: Myoblasten, lymphoiden dendritischen Zellen und Gewebsmakrophagen, rheumatoidem Synovium, Zytokin-stimulierten Neuralzellen, parietalen Epithelzellen der Bowmans Kapsel, dem renalen Tubularepithel, entzündetem Gewebe bei Herz- und Nieren-Transplantat-Abstoßung und von lntestinalgewebe bei Graft versus host-Krankheit. VCAM-1 findet man auch exprimiert auf solchen Gewebearealen des arteriellen Endotheliums, die frühen arteriosklerotischen Plaques eines Kaninchenmodells entsprechen. Zusätzlich wird VCAM-1 auf follikufären dendritischen Zellen von humanen Lymphknoten exprimiert und findet sich auf Stromazellen des Knochenmarks, zum Beispiel in der Maus. Letzterer Befund weist auf eine Funktion von VCAM-1 in der B-Zell-Entwicklung hin. VLA-4 wird, außer auf Zellen haematopoetischen Ursprunges, auch zum Beispiel auf Melanoma-Zellinien gefunden, und der VCAM-1NLA-4-Adhäsionsmechanismus wird mit der Metastasierung von solchen Tumoren in Verbindung gebracht (Rice et al., Science 1989, 246, 1303).

Die hauptsächliche Form, in der VCAM-1 in vivo auf endothelialen Zellen vorkommt und die die dominante Form in vivo ist, wird als VCAM-7D bezeichnet und trägt sieben Immunglobulin-Domänen. Die Domänen 4, 5 und 6 ähneln in ihren Aminosäuresequenzen den Domänen 1, 2 und 3. Die vierte Domäne ist bei einer weiteren, aus sechs Domänen bestehenden Form, hier als VCAM-6D bezeichnet, durch alternatives Splicing entfernt. Auch VCAM-6D kann VLA-4-exprimierende Zellen binden.

Weitere Angaben zu VLA-4, VCAM-1, Integrinen und Adhäsionsproteinen finden sich zum Beispiel in den Artikeln von Kilger und Holzmann, J. Mol. Meth. 1995, 73, 347; Elices, Cell Adhesion in Human Disease, Wiley, Chichester 1995, S. 79; Kuijpers, Springer Semin. Immunopathol. 1995, 16, 379.

Aufgrund der Rolle des VCAM-1 NLA-4-Mechanismus bei Zelladhäsionsprozessen, die von Bedeutung zum Beispiel bei Infektionen, Entzündungen oder Atherosklerose sind, wurde versucht, durch Eingriffe in diese Adhäsionsprozesse Krankheiten zu bekämpfen, insbesondere zum Beispiel Entzündungen (Osborn et al., Cell 1989, 59, 1203). Eine Methode hierzu ist die Verwendung von monoklonalen Antikörpern, die gegen VLA-4 gerichtet sind. Derartige monoklonale Antikörper (mAK), die als VLA-4-Antagonisten die Interaktion zwischen VCAM-1 und VLA-4 blockieren, sind bekannt. So inhibieren zum Beispiel die anti-VLA-4 mAK HP2/1 und HP1/3 die Anheftung von VLA-4 exprimierenden Ramos-Zellen (B-Zell-ähnlichen Zellen) an humane Nabelschnurendothelzellen und an VCAM-1-transfizierte COS-Zellen. Ebenso inhibiert der anti-VCAM-1 mAK 4B9 die Adhäsion von Ramos-Zellen, Jurkat-Zellen (T-Zell-ähnlichen Zellen) und HL60-Zellen (Granulozyten-ähnlichen Zellen) an COS-Zellen transfiziert mit genetischen Konstrukten, die veranlassen, daß VCAM-6D und VCAM-7D exprimiert werden. In vitro-Daten mit Antikörpern, die gegen die α4-Untereinheit von VLA-4 gerichtet sind, zeigen, daß die Anheftung von Lymphozyten an synoviale Endothelzellen blockiert wird, eine Adhäsion, die bei der rheumatoiden Arthritis eine Rolle spielt (van Dinther-Janssen et al., J. Immunol. 1991, 147, 4207).

In vivo-Versuche haben gezeigt, daß eine experimentelle autoimmune Enzephalomyelitis durch anti-a4 mAK gehemmt werden kann. Die Wanderung von Leukozyten in einen Entzündungsherd wird ebenfalls durch einen monoklonalen Antikörper gegen die α4-Kette von VLA-4 blockiert. Die Beeinflussung des VLA-4-abhängigen Adhäsionsmechanismus mit Antikörpern wurde auch in einem Asthma-Modell untersucht, um die Rolle von VLA-4 bei der Rekrutierung von Leukozyten in entzündetes Lungengewebe zu untersuchen (WO-A-93/13798). Die Gabe von anti-VLA-4-Antikörpern inhibierte die Spätphasenreaktion und die Atemwegsüberreaktion in allergischen Schafen.

Der VLA-4 abhängige Zelladhäsionsmechanismus wurde ebenfalls in einem Primatenmodell der inflammatory bowel disease (IBD) untersucht. In diesem Modell, das der ulcerativen Colitis im Menschen entspricht, ergab die Gabe von anti-VLA-4-Antikörpern eine signifikante Reduktion der akuten Entzündung.

Darüber hinaus konnte gezeigt werden, daß die VLA-4-abhängige Zelladhäsion bei den folgenden klinischen Konditionen einschließlich der folgenden chronischen entzündlichen Prozesse eine Rolle spielt: Rheumatoide Arthritis (Cronstein und Weismann, Arthritis Rheum. 1993, 36, 147; Elices et al., J. Clin. Invest. 1994, 93, 405), Diabetes mellitus (Yang et al., Proc. Natl. Acad. Sci. USA 1993, 90, 10494), systemischer Lupus erythematosus (Takeuchi et al., J. Clin. Invest. 1993, 92, 3008), Allergien vom verzögerten Typ (Typ IV-Allergie) (Elices et al., Clin. Exp. Rheumatol. 1993, 11, S77), multiple Sklerose (Yednock et al., Nature 1992, 356, 63), Malaria (Ockenhouse et al., J. Exp. Med. 1992, 176, 1183), Arteriosklerose (O'Brien et al., J. Clin. Invest. 1993, 92, 945), Transplantation (Isobe et al., Transplantation Proceedings 1994, 26, 867-868), verschiedene Malignitäten, zum Beispiel Melanom (Renkonen et al., Am. J. Pathol. 1992, 140, 763), Lymphom (Freedman et al., Blood 1992, 79, 206) und andere (Albelda et al., J. Cell Biol. 1991, 114, 1059).

Eine VLA-4-Blockierung durch geeignete Antagonisten bietet danach effektive therapeutische Möglichkeiten, insbesondere zum Beispiel verschiedene entzündliche Konditionen einschließlich Asthma und IBD zu behandeln. Die besondere Relevanz von VLA-4-Antagonisten für die Behandlung der rheumatoiden Arthritis ergibt sich dabei, wie bereits gesagt, aus der Tatsache, daß Leukozyten aus dem Blut zunächst an endotheliale Zellen anheften müssen, ehe sie in das Synovium auswandern können, und daß bei dieser Anheftung der VLA-4-Rezeptor eine Rolle spielt. Darauf, daß durch Entzündungsagenzien auf endothelialen Zellen VCAM-1 induziert wird (Osborn, Cell 1990, 62, 3; Stoolman, Cell 1989, 56, 907), und auf die Rekrutierung verschiedener Leukozyten in Infektionsgebiete und Entzündungsherde wurde bereits oben eingegangen. T-Zellen adherieren dabei an aktiviertes Endothel hauptsächlich über die LFA-1/ICAM-1- und VLA-4/VCAM-1-Adhäsionsmechanismen (Springer, Cell 1994, 76, 301). Auf den meisten synovialen T-Zellen ist die Bindungskapazität von VLA-4 für VCAM-1 bei der rheumatoiden Arthritis erhöht (Postigo et al., J. Clin. Invest. 1992, 89, 1445). Zusätzlich wurde eine verstärkte Anheftung von synovialen T-Zellen an Fibronektin beobachtet (Laffon et al., J. Clin. Invest. 1991, 88, 546; Morales-Ducret et al., J. Immunol. 1992, 149, 1424). VLA-4 ist also hochreguliert sowohl im Rahmen seiner Expression als auch hinsichtlich seiner Funktion auf T-Lymphozyten der rheumatoiden Synovialmembran. Die Blockierung der Bindung von VLA-4 an seine physiologischen Liganden VCAM-1 und Fibronektin ermöglicht eine effektive Verhinderung oder Linderung von artikulären Entzündungsprozessen. Dies wird auch durch Experimente mit dem Antikörper HP2/1 an Lewis-Ratten mit Adjuvanz-Arthritis bestätigt, bei denen eine effektive Krankheitsprävention beobachtet wurde (Barbadillo et al., Springer Semin. lmmunopathol. 1995, 16, 427). VLA-4 stellt also ein wichtiges therapeutisches Zielmolekül dar.

Die oben erwähnten VLA-4-Antikörper und der Einsatz von Antikörpern als VLA-4-Antagonisten sind in den Patentanmeldungen WO-A-93/13798, WO-A-93/15764, WO-A-94/16094, WO-A-94/17828 und WO-A-95119790 beschrieben. In den Patentanmeldungen WO-A-94/15958, WO-A-95/15973, WO-A-96/00581, WO-A-96/06108 und WO-A-96/20216 werden peptidische Verbindungen als VLA-4-Antagonisten beschrieben. Der Einsatz von Antikörpern und peptidischen Verbindungen als Arzneimitteln ist aber mit Nachteilen behaftet, zum Beispiel mangelnder oraler Verfügbarkeit, leichter Abbaubarkeit oder immunoger Wirkung bei längerfristiger Anwendung, und es besteht somit Bedarf nach VLA-4-Antagonisten mit einem günstigen Eigenschaftsprofil für einen Einsatz in der Therapie und Prophylaxe.

In der WO-A-95/14008, der WO-A-94/21607 (US-A-5 658 935), der WO-A-93/18057, der EP-A-449 079, der EP-A-530 505 (US-A-5 389 614), der EP-A-566 919 (US-A-5 397 796), der EP-A-580 008 (US-A-5 424 293) und der EP-A-584 694 (US-A-5 554 594) sind substituierte 5-Ring-Heterocyclen beschrieben, die am N-terminalen Ende des Moleküls eine Amino-, Amidino- oder Guanidinofunktion aufweisen und die thrombozytenaggregationshemmende Wirkungen zeigen. In der EP-A-796 855 sind weitere Heterocyclen beschrieben, die Inhibitoren der Knochenresorption sind. In der EP-A-842 943 (deutsche Patentanmeldung 19647380.2), der EP-A-842 945 (deutsche Patentanmeldung 19647381.0) und der EP-A-842 944 (deutsche Patentanmeldung 19647382.9) wird beschrieben, daß Verbindungen aus diesen Reihen und weitere Verbindungen überraschenderweise auch die Leukozytenadhäsion hemmen und VLA-4-Antagonisten sind. Hemmstoffe der Leukozytenadhäsion und VLA-4-Antagonisten sind auch in der EP-A-903 353 (deutsche Patentanmeldung 19741235.1), der EP-A-905 139 (deutsche Patentanmeldung 19741873.2) und der EP-A-918 059 (deutsche Patentanmeldung 19751251.8) beschrieben. Weitere Untersuchungen zeigten, daß auch die Verbindungen der vorliegenden Anmeldung starke Hemmstoffe der Leukozytenadhäsion und/oder VLA-4-Antagonisten sind.

Die vorliegende Erfindung betrifft Verbindungen der Formel I, 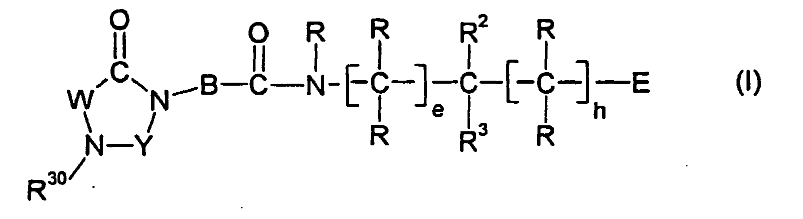 worin
- W: für den zweiwertigen Rest R¹-A-C(R¹³) steht;
- Y: für eine Carbonylgruppe steht;
- B: für einen zweiwertigen Methylenrest oder Ethylenrest steht, wobei der Methylenrest und der Ethylenrest unsubstituiert sind oder substituiert sind durch einen oder zwei gleiche oder verschiedene (C₁-C₈)-Alkylreste;
- E: für R¹⁰CO steht;
- R: für Wasserstoff oder (C₁-C₈)-Alkyl steht, wobei alle Reste R unabhängig voneinander die angegebenen Bedeutungen haben können und gleich oder verschieden sein können;
- R¹-A-: für (C₁-C₄)-Alkyl steht;
- R²: für Wasserstoff oder (C₁-C₈)-Alkyl steht;
- R³: für Wasserstoff, (C₁-C₈)-Alkyl oder gegebenenfalls substituiertes (C₆-C₁₀)-Aryl steht;
- R¹⁰: für Hydroxy oder (C₁-C₆)-Alkoxy steht;
- R¹³: für (C₁-C₄)-Alkyl steht;
- R³⁰: für einen der Reste R³²-CR=CR-R³¹- und R³²-C≡C-R³¹- steht, wobei die Reste R unabhängig voneinander die angegebenen Bedeutungen haben können und gleich oder verschieden sein können;
- R³¹: für den zweiwertigen Rest -R³³-R³⁴-R³⁵-R³⁶-steht, wobei R³⁶ an das Stickstoffatom im Imidazolidinring in der Formel I gebunden ist;
- R³²: für gegebenenfalls substituiertes (C₆-C₁₀)-Aryl oder im Arylrest gegebenenfalls substituiertes (C₆-C₁₀)-Aryl-(C₁-C₄)-alkyl steht;
- R³³: für eine direkte Bindung oder (C₁-C₂)-Alkylen steht;
- R³⁴: für einen zweiwertigen, gegebenenfalls substituierten (C₆-C₁₀)-Arylenrest steht;
- R³⁵: für eine direkte Bindung oder (C₁-C₄)-Alkylen steht;
- R³⁶: für eine direkte Bindung steht;
- e und h: unabhängig voneinander für 0 oder 1 stehen und gleich oder verschieden sein können;
in allen ihren stereoisomeren Formen und Mischungen davon in allen Verhältnissen, und ihre physiologisch verträglichen Salze.

Reste, die mehrfach in den Verbindungen der Formel I auftreten können, können in allen Fällen unabhängig voneinander die angegebenen Bedeutungen haben und gleich oder verschieden sein.

Alkylreste können geradkettig oder verzweigt sein. Dies gilt auch, wenn sie Substituenten tragen oder als Substituenten in anderen Resten auftreten, beispielsweise in Alkoxyresten, Alkoxycarbonylresten oder Arylalkylresten. Von Alkylresten abgeleitete zweiwertige Reste, das heißt Alkylenreste (= Alkandiylreste), können ebenfalls geradkettig oder verzweigt sein. Beispiele für geeignete Alkylreste sind Methyl, Ethyl, n-Propyl, n-Butyl, n-Pentyl, n-Hexyl, n-Heptyl, n-Octyl, Isopropyl, Isobutyl, Isopentyl, Isohexyl, 3-Methylpentyl, Neopentyl, Neohexyl, 2,3,5-Trimethylhexyl, sec-Butyl, tert-Butyl, tert-Pentyl. Bevorzugte Alkylreste sind Methyl, Ethyl, n-Propyl, Isopropyl, n-Butyl, Isobutyl, sec-Butyl, tert-Butyl, n-Pentyl, Isopentyl, n-Hexyl und lsohexyl. Beispiele für Alkylenreste sind die den vorstehend genannten einwertigen Resten entsprechenden zweiwertigen Reste, zum Beispiel Methylen, Ethylen, Trimethylen, Tetramethylen, durch Alkylreste substituiertes Methylen oder Ethylen, zum Beispiel Methylen, das durch eine Methylgruppe, eine Ethylgruppe, eine n-Propylgruppe, eine Isopropylgruppe, eine n-Butylgruppe, eine lsobutylgruppe, eine tert-Butylgruppe, eine n-Pentylgruppe, eine Isopentylgruppe oder eine n-Hexylgruppe substituiert ist, oder zum Beispiel Ethylen, das sowohl an dem einem Kohlenstoffatom als auch an dem anderen Kohlenstoffatom oder auch an an beiden Kohlenstoffatomen substituiert sein kann.

(C₆-C₁₀)-Arylgruppen sind beispielsweise 1-Naphthyl, 2-Naphthyl und Phenyl. Phenylreste sind bevorzugte Arylreste. Arylreste, insbesondere Phenylreste, können unsubstituiert sein oder einfach oder mehrfach, bevorzugt einfach, zweifach oder dreifach, durch gleiche oder verschiedene Reste substituiert sein. Substituierte Reste sind bevorzugt substituiert durch Reste aus der Reihe (C₁-C₈)-Alkyl, insbesondere (C₁-C₄)-Alkyl, (C₁-C₈)-Alkoxy, insbesondere (C₁-C₄)-Alkoxy, Halogen, Nitro, Amino, Trifluormethyl, Hydroxy, Hydroxy-(C₁-C₄)-alkyl wie zum Beispiel Hydroxymethyl oder 1-Hydroxyethyl oder 2-Hydroxyethyl, Methylendioxy, Ethylendioxy, Formyl, Acetyl, Cyano, Hydroxycarbonyl, Aminocarbonyl, (C₁-C₄)-Alkoxycarbonyl, Phenyl, Phenoxy, Benzyl, Benzyloxy und Tetrazolyl. Entsprechendes gilt beispielsweise für Reste wie Arylalkyl Arylalkylreste sind insbesondere Benzyl sowie 1- und 2-Naphthylmethyl, die auch substituiert sein können. Substituierte Arylalkylreste sind beispielsweise durch einen oder mehrere (C₁-C₈)-Alkylreste, insbesondere (C₁-C₄)-Alkylreste, im Arylteil substituierte Benzylreste und Naphthylmethylreste, zum Beispiel 2-, 3- und 4-Methylbenzyl, 4-Isobutylbenzyl, 4-tert-Butylbenzyl, 4-Octylbenzyl, 3,5-Dimethylbenzyl, Pentamethylbenzyl, 2-, 3-, 4-, 5-, 6-, 7- und 8-Methyl-1-naphthylmethyl, 1-, 3-, 4-, 5-, 6-, 7- und 8-Methyl-2-naphthylmethyl; durch einen oder mehrere (C₁-C₈)-Alkoxyreste, insbesondere (C₁-C₄)-Alkoxyreste, im Arylteil substituierte Benzylreste und Naphthylmethylreste, zum Beispiel 4-Methoxybenzyl, 4-Neopentyloxybenzyl, 3,5-Dimethoxybenzyl, 3,4-Methylendioxybenzyl, 2,3,4-Trimethoxybenzyl; Nitrobenzylreste, zum Beispiel 2-, 3- und 4-Nitrobenzyl; Halobenzylreste, zum Beispiel 2-, 3- und 4-Chlor- und 2-, 3-, und 4-Fluorbenzyl, 3,4-Dichlorbenzyl, Pentafluorbenzyl; Trifluormethylbenzylreste, zum Beispiel 3- und 4-Trifluormethylbenzyl oder 3,5-Bis(trifluormethyl)benzyl. Substituierte Arylalkylreste können aber auch unterschiedliche Substituenten aufweisen. In den Verbindungen der Formel I können aber im allgemeinen nicht mehr als zwei Nitrogruppen im Molekül vorhanden sein.

In monosubstituierten Phenylresten kann sich der Substituent in der 2-Position, der 3-Position oder der 4-Position befinden. Zweifach substituiertes Phenyl kann in 2,3-Position, 2,4-Position, 2,5-Position, 2,6-Position, 3,4-Position oder 3,5-Position substituiert sein. In dreifach substituierten Phenylresten können sich die Substituenten in 2,3,4-Position, 2,3,5-Position, 2,4,5-Position, 2,4,6-Position, 2,3,6-Position oder 3,4,5-Position befinden.

Die Erläuterungen zu den Arylresten gelten entsprechend für zweiwertige Arylenreste, zum Beispiel für Phenylenreste, die beispielsweise als 1,4-Phenylen oder als 1,3-Phenylen vorliegen können.

Halogen steht für Fluor, Chlor, Brom oder Iod, insbesondere für Fluor oder Chlor.

Als Beispiele für Substituenten, die der für B stehende Methylenrest oder Ethylenrest tragen kann, seien insbesondere genannt Methyl, Ethyl, n-Propyl, n-Butyl, n-Pentyl, n-Hexyl, n-Heptyl, n-Octyl, Isopropyl, Isobutyl, Isopentyl, Isohexyl, sec-Butyl, tert-Butyl, tert-Pentyl, Neopentyl, Neohexyl, 3-Methylpentyl, 2-Ethylbutyl.

Funktionelle Gruppen in Verbindungen der Formel I können in geschützter Form vorliegen. Geeignete Schutzgruppen wie zum Beispiel Urethanschutzgruppen, Carboxylschutzgruppen und Seitenkettenschutzgruppen sind bei Hubbuch, Kontakte (Merck) 1979, Nr. 3, Seiten 14 bis 23, und bei Büllesbach, Kontakte (Merck) 1980, Nr. 1, Seiten 23 bis 35, beschrieben. Insbesondere seien genannt: Aloc, Pyoc, Fmoc, Tcboc, Z, Boc, Ddz, Bpoc, Adoc, Msc, Moc, Z(NO₂), Z(Halₙ), Bobz, Iboc, Adpoc, Mboc, Acm, tert-Butyl, OBzl, ONbzl, OMbzl, Bzl, Mob, Pic, Trt.

Physiologisch verträgliche Salze der Verbindungen der Formel I sind insbesondere pharmazeutisch verwendbare oder nicht-toxische Salze. Von Verbindungen der Formel I, welche saure Gruppen, zum Beispiel Carbonsäuregruppen enthalten, sind solche Salze beispielsweise Alkalimetallsalze oder Erdalkalimetallsalze, wie zum Beispiel Natriumsalze, Kaliumsalze, Magnesiumsalze und Calciumsalze, sowie Salze mit physiologisch verträglichen quartären Ammoniumionen und Säureadditionssalze mit Ammoniak und physiologisch verträglichen organischen Aminen, wie zum Beispiel Triethylamin, Ethanoiamin,Tris-(2-hydroxyethyl)-amin, α,α,α-Tris-(hydroxymethyl)-methylamin oder Aminosäuren, insbesondere basischen Aminosäuren.

Verbindungen der Formel I, welche basische Gruppen, zum Beispiel eine Aminogruppe enthalten, bilden Salze mit anorganischen Säuren, wie zum Beispiel Salzsäure, Schwefelsäure oder Phosphorsäure, und mit organischen Carbonsäuren oder Sulfonsäuren, wie zum Beispiel Essigsäure, Citronensäure, Benzoesäure, Maleinsäure, Fumarsäure, Weinsäure, Methansulfonsäure oder p-Toluolsulfonsäure. Verbindungen, die sowohl saure Gruppen als auch basische Gruppen enthalten, können auch in Form von inneren Salzen oder Betainen vorliegen, die ebenso von der vorliegenden Erfindung umfaßt werden.

Salze können aus den Verbindungen der Formel I nach üblichen, dem Fachmann bekannten Verfahren erhalten werden; beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen.

Die Verbindungen der Formel **I** können in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren der Verbindungen der Formel I, zum Beispiel Enantiomere und Diastereomere, und Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als linksdrehende als auch als rechtsdrehende Antipoden, in Form von Racematen und in Form von Mischungen der beiden Enantiomeren in allen Verhältnissen Gegenstand der Erfindung. Ebenso sind Diastereomere in reiner Form und in Form von Mischungen von zwei oder mehr Diastereomeren in allen Verhältnissen Gegenstand der Erfindung. Bei Vorliegen einer cis/trans-Isomerie, zum Beispiel an Doppelbindungen, sind sowohl die cis-Form als auch die trans-Form und Mischungen dieser Formen in allen Verhältnissen Gegenstand der Erfindung. Die Herstellung von einzelnen Stereoisomeren kann gewünschtenfalls durch Verwendung von stereochemisch einheitlichen Ausgangssubstanzen bei der Synthese, durch stereoselektive Synthese oder durch Auftrennung eines Gemisches nach üblichen Methoden, zum Beispiel durch Chromatographie oder Kristallisation, erfolgen, im Fall von Enantiomeren zum Beispiel durch Chromatographie an chiralen Phasen. Gegebenenfalls kann vor einer Trennung von Stereoisomeren eine Derivatisierung erfolgen. Die Trennung eines Stereoisomerengemisches kann auf der Stufe der Verbindungen der Formel I erfolgen oder auf der Stufe einer Ausgangssubstanz oder eines Zwischenprodukts im Verlaufe der Synthese.

Die erfindungsgemäßen Verbindungen der Formel I können darüber hinaus bewegliche Wasserstoffatome enthalten, also in verschiedenen tautomeren Formen vorliegen. Auch alle diese Tautomeren sind Gegenstand der vorliegenden Erfindung. Die vorliegende Erfindung umfaßt weiterhin Solvate von Verbindungen der Formel I, zum Beispiel Hydrate und Addukte mit Alkoholen, Derivate von Verbindungen der Formel I, zum Beispiel Ester, Prodrugs und andere physiologisch verträgliche Derivate, sowie aktive Metabolite von Verbindungen der Formel I. Gegenstand der Erfindung sind insbesondere Prodrugs der Verbindungen der Formel I, die unter physiologischen Bedingungen in Verbindungen der Formel I umgewandelt werden. Geeignete Prodrugs für die Verbindungen der Formel I, also chemisch modifizierte Derivate der Verbindungen der Formel I mit in gewünschter Weise verbesserten Eigenschaften, sind dem Fachmann bekannt. Nähere Angaben zu Prodrugs finden sich zum Beispiel in Fleisher et al., Advanced Drug Delivery Reviews 19 (1996) 115-130; Design of Prodrugs, H. Bundgaard, Ed., Elsevier, 1985; H. Bundgaard, Drugs of the Future 16 (1991) 443; Saulnier et al., Bioorg. Med. Chem. Lett. 4 (1994) 1985; Safadi et al., Pharmaceutical Res. 10 (1993) 1350. Als Prodrugs für die Verbindungen der Formel I kommen speziell in Betracht Ester-Prodrugs von Carbonsäuregruppen sowie Acyl-Prodrugs und Carbamat-Prodrugs von acylierbaren stickstoffhaltigen Gruppen wie Aminogruppen.

Die einzelnen Strukturelemente in der Formel I haben bevorzugt die folgenden Bedeutungen, die sie unabhängig voneinander haben können.

B steht bevorzugt für einen zweiwertigen Methylenrest oder Ethylenrest (= 1,2-Ethylen), wobei der Methylenrest und der Ethylenrest unsubstituiert sind oder substituiert sind. Besonders bevorzugt steht B für einen substituierten Methylenrest oder Ethylenrest, insbesondere für einen substituierten Methylenrest. Ist ein für B stehender Methylenrest oder Ethylenrest substituiert, so ist er bevorzugt substituiert durch einen oder zwei gleiche oder verschiedene (C₁-C₈)-Alkylreste, insbesondere durch einen (C₁-C₈)-Alkylrest, also durch geradkettige oder verzweigte Alkylreste mit 1, 2, 3, 4, 5, 6, 7 oder 8 Kohlenstoffatomen.

Die Reste R stehen bevorzugt unabhängig voneinander für Wasserstoff, Methyl oder Ethyl, speziell für Wasserstoff.

R² steht bevorzugt für Wasserstoff oder (C₁-C₆)-Alkyl, besonders bevorzugt für Wasserstoff, Methyl oder Ethyl, speziell für Wasserstoff.

Bevorzugt steht R³ für Wasserstoff, (C₁-C₄)-Alkyl, oder gegebenenfalls substituiertes (C₆-C₁₀)-Aryl.

R¹⁰ steht bevorzugt für Hydroxy oder (C₁-C₄)-Alkoxy.

R¹³ steht bevorzugte für Methyl.

R³³ steht bevorzugt für eine direkte Bindung.

R³⁵ steht bevorzugt für eine direkte Bindung oder (C₁-C₂)-Alkylen, insbesondere eine direkte Bindung oder Methylen oder Ethylen (1,2-Ethylen), ganz besonders bevorzugt für (C₁-C₂)-Alkylen.

R³¹ steht bevorzugt für den zweiwertigen Rest -R³³-R³⁴-R³⁵-R³⁶-, in dem einer oder mehrere der Reste R³³, R³⁴, R³⁵ und R³⁶ bevorzugte Bedeutungen haben.

Bevorzugt steht R³¹ für einen zweiwertigen Rest aus der Reihe gegebenenfalls substituiertes (C₆-C₁₀)-Arylen und im Arylrest gegebenenfalls substituiertes (C₆-C₁₀)-Arylen-(C₁-C₄)-alkyl, wobei im Falle des Arylenalkylrestes die Alkylgruppe an das Stickstoffatom im Imidazolidinring in der Formel I gebunden ist.

e steht bevorzugt für 0 und h für 1.

Bevorzugte Verbindungen der Formel I sind solche Verbindungen, in denen einer oder mehrere der Reste bevorzugte Bedeutungen haben, wobei auch alle Kombinationen von einer oder mehreren bevorzugten Bedeutungen oder von spezifischen Bedeutungen von Resten von der vorliegenden Erfindung umfaßt werden.

Eine Reihe von speziell bevorzugten Verbindungen umfaßt solche Verbindungen der Formel I**,** worin B für unsubstituiertes Methylen steht oder für Methylen steht, das durch einen (C₁-C₈)-Alkylrest substituiert ist, in allen ihren stereoisomeren Formen und Mischungen davon in allen Verhältnissen, und ihre physiologisch verträglichen Salze. Besonders speziell bevorzugt in dieser Reihe sind Verbindungen der Formel I, worin B für Methylen steht, das durch einen (C₁-C₈)-Alkylrest substituiert ist, in allen ihren stereoisomeren Formen und Mischungen davon in allen Verhältnissen, und ihre physiologisch verträglichen Salze.

Eine weitere Reihe von speziell bevorzugten Verbindungen umfaßt solche Verbindungen der Formel I, worin R¹³ für Methyl steht, in allen ihren stereoisomeren Formen und Mischungen davon in allen Verhältnissen, und ihre physiologisch verträglichen Salze. Besonders speziell bevorzugt sind in dieser Reihe Verbindungen der Formel I, worin die Gruppe R¹-A- die Bedeutung Methyl hat.

Eine weitere Reihe von speziell bevorzugten Verbindungen umfaßt solche Verbindungen der Formel I, worin in dem Rest -N(R)-(C(R)(R))ₑ-C(R²)(R³)-(C(R)(R))ₕ-E, der durch eine Amidbindung mit der Gruppe -B-CO- verknüpft ist, die Kette von Kohlenstoffatomen zwischen der Gruppe N(R) und der ersten an diese Kette gebundenen Gruppe, die eine Carbonsäuregruppe oder ein Derivat davon wie einen Ester darstellt, zwei oder mehr als zwei Kohlenstoffatome enthält, in allen ihren stereoisomeren Formen und Mischungen davon in allen Verhältnissen, und ihre physiologisch verträglichen Salze. Diese erste Säuregruppe (oder das Derivat davon), die, ausgehend von der Gruppe N(R), an diese Kette von Kohlenstoffatomen gebunden ist, wird durch die Gruppe E dargestellt. Besonders speziell bevorzugt sind in dieser Reihe Verbindungen der Formel I, worin in dem Rest -N(R)-(C(R)(R))ₑ-C(R²)(R³)-(C(R)(R))ₕ-E die Kette von Kohlenstoffatomen zwischen der Gruppe N(R) und der ersten an diese Kette gebundenen Gruppe, die eine Säuregruppe oder ein Derivat davon darstellt, gerade zwei Kohlenstoffatome umfaßt, in allen ihren stereoisomeren Formen und Mischungen davon in allen Verhältnissen, und ihre physiologisch verträglichen Salze. Derartige besonders speziell bevorzugte Verbindungen der Formel I können zum Beispiel Verbindungen sein, worin e für 1 steht, das heißt Verbindungen, die die Gruppe -N(R)-C(R)(R)-C(R²)(R³)-(C(R)(R))ₕ-E enthalten, wobei im Falle dieser Verbindungen h für 1 oder 0 steht, in allen ihren stereoisomeren Formen und Mischungen davon in allen Verhältnissen, und ihre physiologisch verträglichen Salze. Derartige besonders speziell bevorzugte Verbindungen der Formel I können zum Beispiel auch Verbindungen sein, worin e für 0 steht, h für 1 steht und R³ nicht für eine Säuregruppe oder ein Derivat davon steht, in allen ihren stereoisomeren Formen und Mischungen davon in allen Verhältnissen, und ihre physiologisch verträglichen Salze, das heißt Verbindungen, die einen Rest -N(R)-C(R²)(R^{3a})-C(R)(R)-E enthalten, worin R^{3a} wie R³ definiert ist. Bevorzugt ist es in den Verbindungen dieser Reihe weiterhin, wenn die Gruppe -N(R)- im Rest -N(R)-(C(R)(R))ₑ-C(R²)(R³)-(C(R)(R))ₕ-E für die Gruppe -NH- steht.

Eine weitere Reihe von speziell bevorzugten Verbindungen umfaßt solche Verbindungen der Formel I**,** worin in dem Rest -N(R)-(C(R)(R))ₑC(R²)(R³)-(C(R)(R))ₕ-E die Kette von Kohlenstoffatomen zwischen der Gruppe N(R) und der ersten an diese Kette gebundenen Gruppe, die eine Säuregruppe oder ein Derivat davon darstellt, nur ein Kohlenstoffatom umfaßt, in allen ihren stereoisomeren Formen und Mischungen davon in allen Verhältnissen und ihre physiologisch verträglichen Salze, wobei aber in diesen Verbindungen die erste Säuregruppe oder das Derivat davon, das ausgehend von der Gruppe N(R) an die Kette von Kohlenstoffatomen gebunden ist, die folgende Bedingung erfüllen muß: die erste Säuregruppe ist eine freie Säuregruppe (oder ein Salz davon), oder die erste Säuregruppe oder das Derivat davon ist eine Estergruppe.

Generell sind Verbindungen der Formel I bevorzugt, die an Chiralitätszentren, zum Beispiel bei entsprechender Substitution an dem die Reste R² und R³ tragenden Kohlenstoffatom oder an dem Zentrum W im Imidazolidin-Ring in der Formel I, eine einheitliche Konfiguration aufweisen, wobei die einzelnen Chiralitätszentren unabhängig voneinander die R-Konfiguration oder die S-Konfiguration aufweisen können.

Die Verbindungen der Formel I können beispielsweise hergestellt werden durch Fragmentkondensation einer Verbindung der Formel II 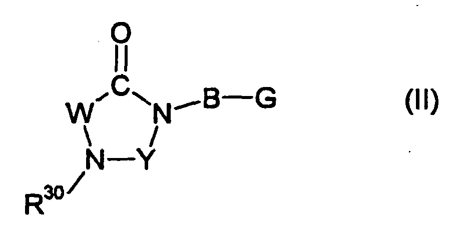 mit einer Verbindung der Formel III, 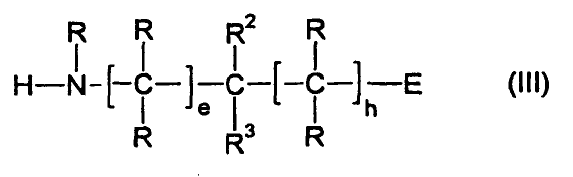 wobei in den Formeln II und III die Gruppen W, Y, B, E, R, R², R³, R³⁰ sowie e und h wie oben angegeben definiert sind oder auch in diesen Gruppen funktionelle Gruppen in geschützter Form oder in Form von Vorstufen enthalten sein können, und wobei G für Hydroxycarbonyl, (C₁-C₆)-Alkoxycarbonyl oder aktivierte Carbonsäurederivate wie Säurechloride oder Aktivester steht.

Zur Kondensation der Verbindungen der Formel II mit denen der Formel III verwendet man vorteilhafterweise die dem Fachmann an sich wohlbekannten Kupplungsmethoden der Peptidchemie (siehe zum Beispiel Houben-Weyl, Methoden der Organischen Chemie, Band 15/1 und 15/2, Georg Thieme Verlag, Stuttgart, 1974). Als Kondensationsmittel bzw. Kupplungsreagenzien kommen zum Beispiel Carbonyldiimidazol, Carbodiimide wie Dicyclohexylcarbodiimid oder Diisopropylcarbodiimid, das O-((Cyano(ethoxycarbonyl)methylen)amino)-N,N,N',N'-tetramethyluronium-tetrafluoroborat (TOTU) oder Propylphosphonsäureanhydrid (PPA) in Frage.

Die Kondensationen können unter wohlbekannten Standardbedingungen durchgeführt werden. Bei der Kondensation ist es in der Regel nötig, daß vorhandene, nicht reagierende Aminogruppen durch reversible Schutzgruppen geschützt werden. Gleiches gilt für nicht an der Reaktion beteiligte Carboxylgruppen, die während der Kondensation bevorzugt als (C₁-C₆)-Alkylester, Benzylester oder tert-Butylester vorliegen. Ein Aminogruppen-Schutz erübrigt sich, wenn die Aminogruppen noch in Form von Vorstufen, zum Beispiel als Nitrogruppen oder Cyanogruppen, vorliegen und erst nach der Kondensation zum Beispiel durch Hydrierung gebildet werden. Nach der Kondensation werden die vorhandenen Schutzgruppen in geeigneter Weise abgespalten. Beispielsweise können Benzyloxycarbonylgruppen und Benzylgruppen in Benzylestern abhydriert werden. Die Schutzgruppen vom tert-Butyltyp werden sauer abgespalten, während der 9-Fluorenylmethyloxycarbonylrest durch sekundäre Amine entfernt wird. Die Herstellung der Verbindungen der Formel I kann beispielsweise auch erfolgen, indem man die Verbindungen nach üblichen Methoden schrittweise an einer Festphase aufbaut, wobei die einzelnen Bauelemente des Moleküls in unterschiedlicher Reihenfolge eingeführt werden können.

Verbindungen der Formel II können beispielsweise hergestellt werden, indem man zunächst Verbindungen der Formel IV 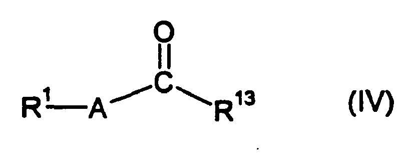 in einer Bucherer-Reaktion zu Verbindungen der Formel V 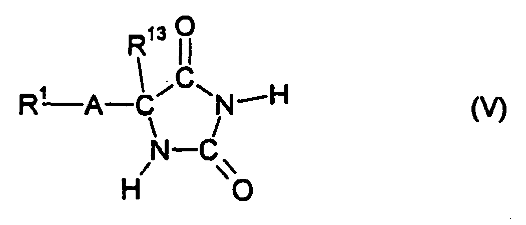 umsetzt (H. T. Bucherer, V. A. Lieb, J. Prakt. Chem. 141 (1934), 5), wobei in den Formeln IV und V die Gruppen R¹, R¹³ und A wie oben angegeben definiert sind. Verbindungen der Formel VI, 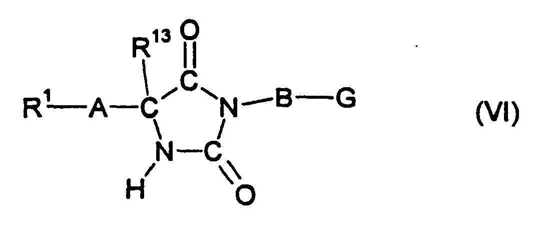 in der R¹, R¹³, A, B und G wie oben angegeben definiert sind, können dann erhalten werden, indem man die Verbindungen der Formel V beispielsweise zunächst mit einem alkylierenden Reagenz umsetzt, das den Rest -B-G in das Molekül einführt. Die Umsetzung von Verbindungen der Formel VI mit einem zweiten Reagenz der Formel R³⁰-LG, in der R³⁰ die oben angegebenen Bedeutungen hat und LG eine nucleophil substituierbare Abgangsgruppe, zum Beispiel Halogen, insbesondere Chlor, Brom oder lod, Sulfonyloxy wie Tosyloxy, Methylsulfonyloxy oder Trifluormethylsulfonyloxy, (C₁-C₄)-Alkoxy, gegebenenfalls substituiertes Phenoxy oder eine heterocyclische Abgangsgruppe wie zum Beispiel Imidazolyl, darstellt, führt zu dann den entsprechenden Verbindungen der Formel II.

Generell kann es je nach den Bedeutungen des Restes R³⁰ und anderer Reste auch vorteilhaft sein, nicht den endgültigen Rest R³⁰ mittels des Reagenzes R³⁰-LG in das Molekül einzuführen, sondern nach Anknüpfung einer Vorstufe der Gruppe R³⁰ an den Imidazolidinring den Rest R³⁰ am lmidazolidinring aufzubauen. Dies kann zum Beispiel auf der Stufe einer Verbindung der Formel VI bzw. der daraus hergestellten Verbindung der Formel II erfolgen oder auf der Stufe eines anderen Zwischenprodukts der Synthese. Zum Beispiel kann eine Verbindung der Formel VI mit einer Verbindung der Formel RG-R³¹-LG zu einer Verbindung der Formel VII 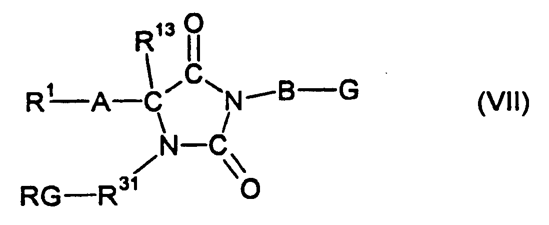 umgesetzt werden, in der A, B, G, R¹, R¹³ und R³¹ die oben angegebenen Bedeutungen haben. In der Formel RG-R³¹-LG steht LG wie oben für eine nucleophil substituierbare Abgangsgruppe, zum Beispiel Halogen, insbesondere Chlor, Brom oder lod, oder einen Sulfonyloxyrest. RG- steht in der Formel RG-R³¹-LG und in der Formel VII für eine reaktive Gruppe, die in einen der Reste R³²-CR=CR-, R³²-C≡C- überführt werden kann. RG kann beispielsweise für Halogen, Cyan oder eine Carbonylgruppe stehen. Die Überführung der Gruppe RG in die gewünschte Zielgruppe kann in einem oder mehreren Schritten erfolgen und nach dem Fachmann geläufigen Standardverfahren durchgeführt werden. Beispielhaft ist diese Vorgehensweise im folgenden für die Herstellung von Verbindungen der Formel II erläutert, in der R³⁰ für R³²-CR=CR- steht.

So können Verbindungen der Formel VI in Verbindungen der Formel VII überführt werden, in denen die Gruppe RG eine Gruppe ist, die eine Vorstufe für eine Aldehydgruppe darstellt und die im nächsten Reaktionsschritt dann in eine Aldehydgruppe überführt wird. Beispielsweise kann eine Verbindung der Formel VI zunächst mit einer Cyanverbindung der Formel NC-R³¹-LG zu einer Verbindung der Formel VII umgesetzt werden, in der RG für Cyan steht. Die Cyangruppe kann dann beispielsweise durch Reduktion in eine Aldehydgruppe überführt werden (zum Beispiel analog D. M. Flanagan und M. M. Joullie, Synthetic Communications 1990, 20, 459-467). Die Aldehydgruppe kann anschließend in einer Horner-Emmons-Reaktion, beispielsweise mit einem Phosphoran der Formel R³²-C(R)=P(O)(OC₂H₅)₂ unter Verwendung einer geeigneten Base wie zum Beispiel Natriumhydrid, oder in einer anderen üblichen Carbonylolefinierungsreaktion zu einer Verbindung der Formel II umgesetzt werden, in der R³⁰ für R³²-CR=CR-R³¹- steht. Gemäß dieser Vorgehensweise können zahlreiche weitere Überführungen in Verbindungen der Formel II durchgeführt werden.

Weiterhin können aus Verbindungen der Formel VI mit einem Reagenz der Formel Hal-R³¹-LG, in der Hal für Halogen, insbesondere Chlor, Brom oder lod, steht, Verbindungen der Formel VII hergestellt werden, in der RG für Halogen steht. Derartige Verbindungen können beispielsweise in einer Heck-Reaktion in Gegenwart eines Palladium(0)-Katalysators mit Olefinen der Formel R³²-CR=CHR, zum Beispiel mit Styrolen, zu Verbindungen der Formel II umgesetzt werden, in der R³⁰ für R³²-CR=CR-R³¹- steht, zum Beispiel zu Stilben-Derivaten (vergleiche R. F. Heck, Org. Reactions 1982, 27, 345). Analog können aus Verbindungen der Formel VII, in der RG für Halogen steht, in einer Heck-Reaktion in Gegenwart eines Palladium(0)-Katalysators mit Acetylenen der Formel R³²-C≡CH, zum Beispiel mit Phenylacetylenen, Verbindungen der Formel II erhalten werden, in der R³⁰ für R³²-C≡C-R³¹- steht, zum Beispiel Tolan-Derivate.

In Verbindungen der Formel II, aber ebenso auch in Verbindungen der Formel I, können Doppelbindungen und Dreifachbindungen in der Gruppe R³⁰ ineinander umgewandelt werden. Verbindungen, in denen R³⁰ für R³²-C≡C-R³¹- steht, können durch partielle Hydrierung in Verbindungen mit einer C-C-Doppelbindung umgewandelt werden. Verbindungen, in denen R³⁰ für R³²-CR=CR-R³¹- steht, können durch Bromierung der Doppelbindung mit elementarem Brom in die entsprechenden Dibromide und anschließen durch Dehydrohalogenierung in Verbindungen überführt werden, in denen R³⁰ für R³²-C≡C-R³¹- steht, zum Beispiel Tolan-Derivate (siehe zum Beispiel G.W. Kabalka, K Yang, N.K Reddy, C. Narayana, Synthetic Communications 1998, 28(5), 925-929; S. Nakatsuji, K Matsuda, Y. Uesugi, K. Nakashima, S. Akiyama und W. Fabian, J. Chem. Soc. Perk. Trans. I 1992, 755 - 758; K Fukunaga und H. Yamaguchi, Synthesis 1981, 879 - 880).

Gemäß diesen Vorgehensweise können zahlreiche weitere Verbindungen der Formel I aufgebaut werden, wobei die durchzuführenden Reaktionen stets Standardverfahren sind, die dem Fachmann geläufig sind.

Ganz generell können die einzelnen Schritte bei der Herstellung der Verbindungen der Formel I mach oder analog zu bekannten, dem Fachmann geläufigen Methoden durchgeführt werden. Je nach dem Einzelfall kann es hierbei, wie bereits erläutert, bei allen Schritten in der Synthese der Verbindungen der Formel I angebracht sein, funktionelle Gruppen, die zu Nebenreaktionen oder unerwünschten Reaktionen führen könnten, durch eine dem Syntheseproblem angepaßte Schutzgruppenstrategie temporär zu blockieren, was dem Fachmann bekannt ist.

Die erläuterte Vorgehensweise, funktionelle Gruppen nicht direkt in der endgültigen Form in das Molekül einzuführen, sondern zunächst Vorstufen in das Molekül einzuführen und dann auf der Stufe eines Zwischenprodukts die endgültige funktionelle Gruppe aufzubauen, kann, wie bereits erwähnt, entsprechend auch für andere Teile der Moleküls der Formel **I** angewandt werden, beispielsweise für die Gruppe R³.

Die. Aminoverbindungen der Formel III sind käuflich oder können nach oder analog zu wohlbekannten Standardverfahren aus Ausgangsverbindungen aufgebaut werden, die käuflich sind oder nach oder analog zu Literaturvorschriften erhältlich sind.

Verbindungen der Formel I können auch wie folgt erhalten werden:
Durch Reaktion von nach Standardverfahren erhältlichen α-Aminosäuren oder N-substituierten α-Aminosäuren oder bevorzugt deren Estern, zum Beispiel der Methylester, Ethylester, tert-Butylester oder Benzylester, beispielsweise von Verbindungen der Formel XI, 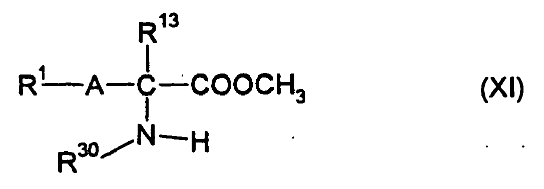
worin R¹, R¹³, R³⁰ und A wie oben angegeben definiert sind, mit einem Isocyanat oder Isothiocyanat beispielsweise der Formel XII, 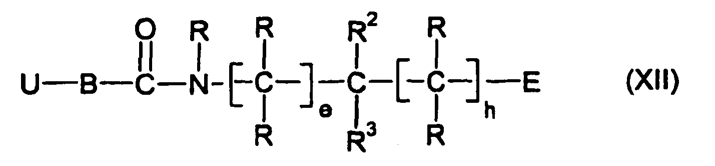 worin B, E, R, R², R³, e und h wie oben angegeben definiert sind und U für Isocyanato steht, erhält man Harnstoffderivate beispielsweise der Formel XIII, 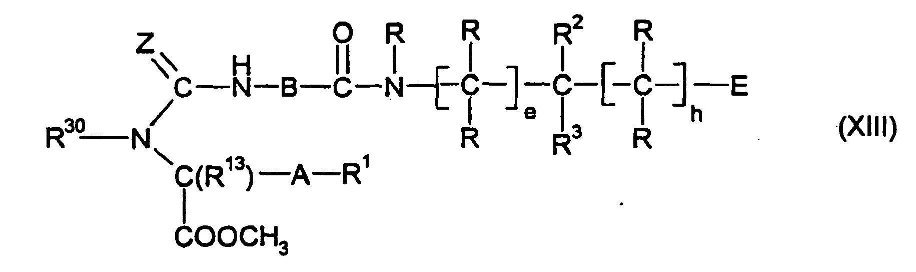 für die die oben angegebenen Definitionen gelten und in der Z für Sauerstoff steht. Die Verbindungen der Formel XIII können durch Erhitzen mit Säure zu Verbindungen der Formel la 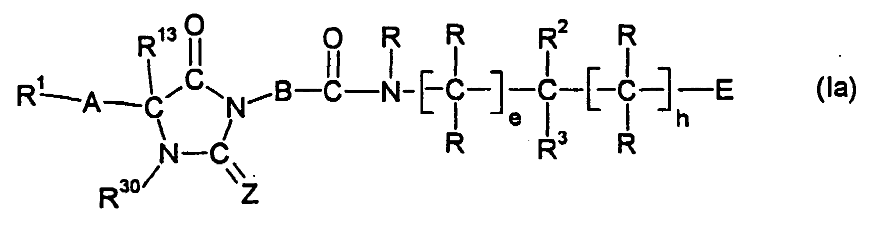 cyclisiert werden, für die die oben angegebenen Bedeutungen gelten. Die Cyclisierung der Verbindungen der Formel XIII zu den Verbindungen der Formel la kann auch durch Behandlung mit Basen in inerten Lösungsmittel durchgeführt werden, zum Beispiel durch Behandlung mit Natriumhydrid in einem aprotischen Lösungsmittel wie Dimethylformamid. Während der Cyclisierung können wiederum funktionelle Gruppen in geschützter Form vorliegen.

Verbindungen der Formel I können auch erhalten werden, indem man eine Verbindung der Formel XI mit einem Isocyanat der Formel XIV 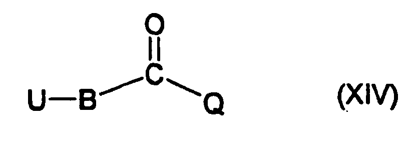 umsetzt, in der B und U wie oben für die Formel XII angegeben definiert sind und Q eine Alkoxygruppe, zum Beispiel eine (C₁-C₄)-Alkoxygruppe wie Methoxy, Ethoxy oder tert-Butoxy, eine (C₆-C₁₄)-Aryloxygruppe, zum Beispiel Phenoxy, oder eine (C₆-C₁₄)-Aryl-(C₁-C₄)-alkoxygruppe, zum Beispiel Benzyloxy, bedeutet. Dabei wird eine Verbindung der Formel XV 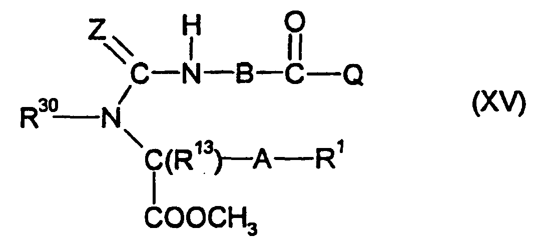 erhalten, in der Z für Sauerstoff steht und A, B, Q, R¹ R¹³ und R³⁰ wie oben für die Formeln XI und XIV angegeben definiert sind, die dann unter dem Einfluß einer Säure oder einer Base, wie oben für die Cyclisierung der Verbindungen der Formel XIII beschrieben, zu einer Verbindung der Formel XVI, 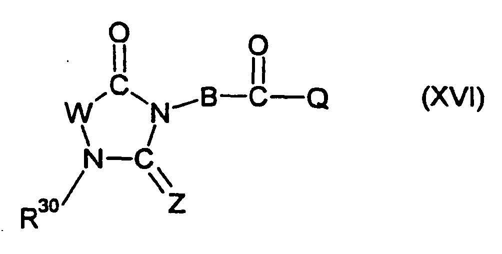 in der W für R¹-A-C(R¹³) steht und Z, B, Q und R³⁰ wie oben angegeben definiert sind, cyclisiert wird. Aus der Verbindung der Formel XVI kann dann durch Hydrolyse der Gruppe CO-Q zur Carbonsäure COOH und nachfolgende Kupplung mit einer Verbindung der Formel III, wie oben für die Kupplung der Verbindungen der Formeln II und III beschrieben, eine Verbindung der Formel la erhalten werden. Auch hier können während der Cyclisierung funktionelle Gruppen in geschützter Form oder in Form von Vorstufen vorliegen. Anstatt von Verbindungen der Formel XI kann auch von analogen Verbindungen ausgegangen werden, die in der Aminogruppe an Stelle der Gruppe R³⁰ ein Wasserstoffatom enthalten, und die Gruppe R³⁰ dann wie erläutert später in einem oder in mehreren Schritten in das Molekül eingeführt werden.

Eine weitere Methode zur Herstellung von Verbindungen der Formel la ist beispielsweise die Umsetzung von Verbindungen der Formel XVII, 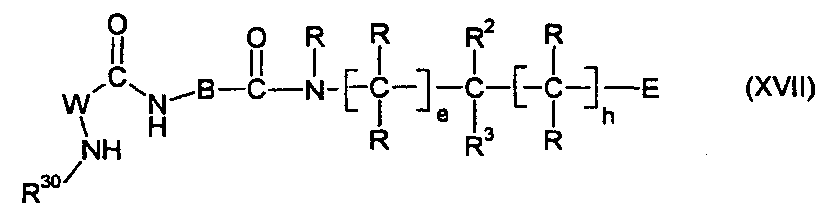 in der W für R¹-A-C(R¹³) steht und für die ansonsten die oben angegebenen Definitionen gelten, mit Phosgen oder entsprechenden Äquivalenten (analog S. Goldschmidt und M. Wick, Liebigs Ann. Chem. 575 (1952), 217-231 und C. Tropp, Chem. Ber. 61 (1928), 1431-1439).

Hinsichtlich der Herstellung der Verbindungen der Formel I wird weiterhin Bezug genommen auf die WO-A-95/14008, auf die EP-A-796 855 und die ihr entsprechenden Anmeldungen, sowie auf die WO-A-96/33976. Insbesondere wird auch hinsichtlich der Herstellung der Verbindungen der Formeln V und VI in racemischer Form und in eriantiomerenreiner Form Bezug genommen auf die entsprechenden Ausführungen in der WO-A-96/33976.

Die Verbindungen der Formel I sind wertvolle Arzneimittelwirkstoffe, die sich beispielsweise für die Therapie und Prophylaxe von Entzündungserkrankungen, allergischen Erkrankungen oder Asthma eignen. Die Verbindungen der Formel I und ihre physiologisch verträglichen Salze können erfindungsgemäß am Tier, bevorzugt am Säugetier, und insbesondere am Menschen als Arzneimittel zur Therapie oder Prophylaxe verabreicht werden. Sie können für sich allein, in Mischungen untereinander oder in Form von pharmazeutischen Präparaten verabreicht werden, die eine enterale oder parenterale Anwendung gestatten und die als aktiven Bestandteil eine wirksame Dosis mindestens einer Verbindung der Formel I und/oder ihrer physiologisch verträglichen Salze neben üblichen pharmazeutisch einwandfreien Trägerstoffen und/oder Zusatzstoffen enthalten.

Gegenstand der vorliegenden Erfindung sind daher auch die Verbindungen der Formel **I** und/oder ihre physiologisch verträglichen Salze zur Verwendung als Arzneimittel, die Verwendung der Verbindungen der Formel I und/oder ihrer physiologisch verträglichen Salze zur Herstellung von Arzneimitteln für die Therapie und Prophylaxe der oben und im folgenden erläuterten Krankheiten, zum Beispiel für die Therapie und Prophylaxe von Entzündungserkrankungen, sowie die Verwendung der Verbindungen der Formel I und/oder ihrer physiologisch verträglichen Salze bei der Therapie und Prophylaxe dieser Krankheiten. Weiterhin sind Gegenstand der vorliegenden Erfindung pharmazeutische Präparate (oder pharmazeutische Zusammensetzungen), die eine wirksame Dosis mindestens einer Verbindung der Formel **I** und/oder ihrer physiologisch verträglichen Salze und einen üblichen pharmazeutisch einwandfreien Träger enthalten.

Die Arzneimittel können systemisch oder lokal verabreicht werden. Sie können zum Beispiel oral in Form von Pillen, Tabletten, Filmtabletten, Dragees, Granulaten, Hart- und Weichgelatinekapseln, Pulvern, Lösungen, Sirupen, Emulsionen, Suspensionen oder in anderen Arzneiformen verabreicht werden. Die Verabreichung kann aber auch vaginal oder rektal, zum Beispiel in Form von Suppositorien, oder parenteral oder implantiv, zum Beispiel in Form von Injektionslösungen oder Infusionslösungen, Mikrokapseln oder Rods, oder topisch oder perkutan, zum Beispiel in Form von Salben, Lösungen oder Tinkturen, oder auf anderem Wege, zum Beispiel in Form von Nasalsprays oder Aerosolmischungen, erfolgen. Parenteral kann die Verabreichung zum Beispiel intravenös, intramuskulär, subkutan, intraartikulär, intrasynovial oder auf andere Weise erfolgen.

Die Herstellung der erfindungsgemäßen pharmazeutischen Präparate erfolgt in an sich bekannter Weise, wobei neben der oder den Verbindungen der Formel I und/oder ihren physiologisch verträglichen Salzen pharmazeutisch inerte anorganische und/oder organische Trägerstoffe verwendet werden können. Für die Herstellung von Pillen, Tabletten, Dragees und Hartgelatinekapseln kann man zum Beispiel Lactose, Maisstärke oder Derivate davon, Talk, Stearinsäure oder deren Salze, etc. verwenden. Trägerstoffe für Weichgelatinekapseln und Suppositorien sind zum Beispiel Fette, Wachse, halbfeste und flüssige Polyole, natürliche oder gehärtete Öle etc. Als Trägerstoffe für die Herstellung von Lösungen, zum Beispiel Injektionslösungen, oder von Emulsionen oder Sirupen eignen sich zum Beispiel Wasser, Alkohole, Diole, Glycerin, Polyole, Saccharose, Invertzucker, Glukose, pflanzliche Öle etc. Als Trägerstoffe für Mikrokapseln, Implantate oder Rods eignen sich zum Beispiel Mischpolymerisate aus Glykolsäure und Milchsäure. Die pharmazeutischen Präparate enthalten normalerweise etwa 0,5 bis 90 Gew.-% der Verbindungen der Formel I und/oder ihrer physiologisch verträglichen Salze. Die Menge an Wirkstoff der Formel I und/oder dessen physiologisch verträglichen Salzen in den pharmazeutischen Präparaten beträgt normalerweise 0,2 bis 500 mg, vorzugsweise 1 bis 200 mg, es können aber auch größere Wirkstoffmengen enthalten sein.

Die pharmazeutischen Präparate können neben den Wirkstoffen und Trägerstoffen noch Zusatzstoffe (oder Hilfsstoffe) enthalten, wie zum Beispiel Füllstoffe, Spreng-, Binde-, Gleit-, Netz-, Stabilisierungs-, Emulgier-, Konservierungs-, Süß-, Färbe-, Geschmacks-, Aromatisierungs-, Dickungs- oder Verdünnungsmittel, Puffersubstanzen, Lösungsmittel, Lösungsvermittler, Mittel zur Erzielung eines Depoteffekts, Salze zur Veränderung des osmotischen Drucks, Überzugsmittel oder Antioxidantien. Sie können auch zwei oder mehrere Verbindungen der Formel I und/oder deren physiologisch verträgliche Salze enthalten. Ferner können sie neben mindestens einer Verbindung der Formel I und/oder ihren physiologisch verträglichen Salzen noch einen oder mehrere andere therapeutisch oder prophylaktisch wirksame Stoffe, zum Beispiel Stoffe mit entzündungshemmender Wirkung, enthalten.

Wenn die Verbindungen der Formel I bzw. sie enthaltende pharmazeutische Zubereitungen als Aerosole verabreicht werden, zum Beispiel in Form von Nasalaerosolen oder durch Inhalation, so kann dies beispielsweise unter Verwendung eines Sprays, eines Zerstäubers, eines Pumpzerstäubers, eines Inhalationsgerätes, eines Dosierinhalators oder eines Trockenpulverinhalätors erfolgen. Arzneiformen für eine Verabreichung der Verbindungen der Formel I als Aerosol können nach dem Fachmann wohlbekannten Verfahren hergestellt werden. In Betracht kommen für deren Herstellung beispielsweise Lösungen oder Dispersionen der Verbindungen der Formel I in Wasser, Wasser-Alkohol-Gemischen oder geeigneten Kochsalzlösungen unter Verwendung von üblichen Zusatzstoffen, zum Beispiel Benzylalkohol oder anderen geeigneten Konservierungsmitteln, Absorptionsverbesserern zur Erhöhung der Bioverfügbarkeit, Lösungsvermittlern, Dispergiermitteln und anderen, und gegebenenfalls üblichen Treibmitteln, zum Beispiel Fluorchlorkohlenwasserstoffen und/oder Fluorkohlenwasserstoffen.

Die Verbindungen der Formel I haben beispielsweise die Fähigkeit, Zell-Zelllnteraktionsprozesse und Zell-Matrix-Interaktionsprozesse zu inhibieren, bei denen Wechselwirkungen zwischen VLA-4 mit seinen Liganden eine Rolle spielen. Die Wirksamkeit der Verbindungen der Formel I kann zum Beispiel in einem Assay nachgewiesen werden, in dem die Bindung von Zellen, die den VLA-4-Rezeptor aufweisen, zum Beispiel von Leukozyten, an Liganden dieses Rezeptors gemessen wird, zum Beispiel an VCAM-1, das dafür vorteilhafterweise auch gentechnisch hergestellt werden kann. Einzelheiten eines solchen Assay sind weiter unten beschrieben. Insbesondere vermögen die Verbindungen der Formel I sie Adhäsion und die Migration von Leukozyten inhibieren, etwa die Anheftung von Leukozyten an endotheliale Zellen, die - wie oben erläutert - über den VCAM-1NLA-4-Adhäsionsmechanismus gesteuert wird. Außer als Entzündungshemmstoffe eignen sich die Verbindungen der Formel I und ihre physiologisch verträglichen Salze daher allgemein zur Therapie und Prophylaxe von Krankheiten, die auf der Wechselwirkung zwischen dem VLA-4-Rezeptor und seinen Liganden beruhen oder durch eine Hemmung dieser Wechselwirkung beeinflußt werden können, und insbesondere eignen sie sich für die Therapie und Prophylaxe von Krankheiten, die zumindest teilweise durch ein unerwünschtes Ausmaß an Leukozytenadhäsion und/oder Leukozytenmigration verursacht werden oder damit verbunden sind, und zu deren Vorbeugung, Linderung oder Heilung die Adhäsion und/oder Migration von Leukozyten verringert werden soll.

Gegenstand der vorliegenden Erfindung sind daher auch die Verbindungen der Formel I zur Hemmung der Adhäsion und/oder Migration von Leukozyten oder zur Hemmung des VLA-4-Rezeptors und die Verwendung der Verbindungen der Formel I zur Herstellung von Arzneimitteln dafür, also von Arzneimitteln zur Therapie oder Prophylaxe von Krankheiten, bei denen die Leukozytenadhäsion und/oder Leukozytenmigration ein unerwünschtes Ausmaß aufweist, oder zur Therapie oder Prophylaxe von Krankheiten, bei denen VLA-4-abhängige Adhäsionsvorgänge eine Rolle spielen, sowie die Verwendung der Verbindungen der Formel I und/oder ihrer physiologisch verträglichen Salze bei der Therapie und Prophylaxe derartiger Krankheiten.

Die Verbindungen der Formel I können bei entzündlichen Erscheinungen unterschiedlichster Ursache als Entzündungshemmer eingesetzt werden, um die unerwünschten oder schädigenden Folgen der Entzündung zu verhindern, zu verringern oder zu unterdrücken. Anwendung finden sie beispielsweise zur Therapie oder Prophylaxe der Arthritis, der rheumatoiden Arthritis, der Polyarthritis, der inflammatory bowel disease (ulcerativen Colitis), des systemischen Lupus erythematosus, zur Therapie oder Prophylaxe von inflammatorischen Erkrankungen des zentralen Nervensystems, wie zum Beispiel der Multiplen Sklerose, oder zur Therapie oder Prophylaxe von Asthma oder von Allergien, zum Beispiel Allergien vom verzögerten Typ (Typ IV-Allergie). Weiterhin eignen sie sich zur Therapie oder Prophylaxe von cardiovaskulären Erkrankungen, der Arteriosklerose, von Restenosen, von Diabetes, der Schädigung von Organtransplantaten, von Immunerkrankungen, von Autoimmunerkrankungen, von Tumorwachstum oder Tumormetastasierung bei verschiedenen Malignitäten, der Malaria sowie von weiteren Krankheiten, bei denen eine Blockierung des Integrins VLA-4 und/oder eine Beeinflussung der Leukozytenaktivität zur Vorbeugung, Linderung oder Heilung angebracht erscheint.

Die Dosis bei der Anwendung der Verbindungen der Formel I kann innerhalb weiter Grenzen variieren und ist wie üblich in jedem einzelnen Fall den individuellen Gegebenheiten anzupassen, was dem Arzt bekannt ist. Sie hängt beispielsweise von der Art und Schwere der zu behandelnden Krankheit ab, von der eingesetzten Verbindung oder davon, ob ein akuter oder chronischer Krankheitszustand behandelt wird oder Prophylaxe betrieben wird, oder davon, ob neben den Verbindungen der Formel I weitere Wirkstoffe verabreicht werden. Im allgemeinen ist bei der oralen Verabreichung eine Tagesdosis von etwa 0,01 bis 100 mg/kg, vorzugsweise 0,1 bis 10 mg/kg, insbesondere 0,3 bis 2 mg/kg (jeweils pro kg Körpergewicht) bei einem ca. 75 kg schweren Erwachsenen zur Erzielung wirksamer Ergebnisse angemessen. Bei intravenöser Applikation beträgt die Tagesdosis im allgemeinen etwa 0,01 bis 50 mg/kg, vorzugsweise 0,01 bis 10 mg/kg Körpergewicht. Die Tagesdosis kann, insbesondere bei der Applikation größerer Mengen, in mehrere, zum Beispiel 2, 3, oder 4, Teilverabreichungen aufgeteilt werden. Gegebenenfalls kann es je nach individuellem Verhalten erforderlich werden, von der angegebenen Tagesdosis nach oben oder nach unten abzuweichen.

Die Verbindungen der Formel I und ihre Salze können weiterhin für diagnostische Zwecke, zum Beispiel bei in vitro-Diagnosen, und als Hilfsmittel in biochemischen Untersuchungen eingesetzt werden, bei denen eine VLA-4-Blockierung oder eine Beeinflussung von Zell-Zell- oder Zell-Matrix-Interaktionen angestrebt wird. Weiterhin können sie als Zwischenprodukte für die Herstellung anderer Verbindungen dienen, insbesondere anderer Arzneimittelwirkstoffe, die aus den Verbindungen der Formel I beispielsweise durch Abwandlung oder Einführung von Resten oder funktionellen Gruppen erhältlich sind.

### Beispiele

Die Produkte wurden über Massenspektren (MS) und/oder NMR-Spektren identifiziert. Basische Verbindungen, die durch Chromatographie unter Verwendung eines Laufmittels gereinigt wurden, das beispielsweise Essigsäure oder Trifluoressigsäure enthielt, und anschließend gefriergetrocknet wurden, oder die mit einer Säure, zum Beispiel mit Trifluoressigsäure, behandelt wurden und zur Aufarbeitung zum Beispiel gefriergetrocknet wurden, enthielten zum Teil je nach Durchführung der Gefriertrocknung oder Aufarbeitung noch die verwendete Säure, sind also teilweise oder vollständig in Form eines Salzes der verwendeten Säure, zum Beispiel in Form des Essigsäuresalzes oder Trifluoressigsäuresalzes, angefallen. Angegebene Mischungsverhältnisse von Lösungsmitteln oder Reagenzien sind Volumenverhältnisse.

Es bedeuten:
- MTBE: Methyl-tert-butylether
- DMF: N,N-Dimethylformamid
- THF: Tetrahydrofuran
- DCC: N,N'-Dicyclohexylcarbodiimid
- TOTU: O-((Cyano(ethoxycarbonyl)methylen)amino)-N,N,N',N'-tetramethyluronium-tetrafluoroborat
- HOBt: 1-Hydroxybenzotriazol
- TFA: Trifluoressigsäure
- Phosphazen P1: tert-Butylimino-tris-(dimethylamino)-phosphoran

Die Verbindungen wurden auf den in den Schemata 1 bis 3 dargestellten Wegen nach den im folgenden beschriebenen allgemeinen Verfahren hergestellt. tBu für tert-Butyl, Alkyl im Schema 1 steht für Methyl oder Ethyl. Im Verfahren gemäß Schema 1 wurde zur Herstellung des Zwischenprodukts der Formel VIa ein in der α-Position durch die Gruppen R¹³ und R¹-A- substituierter α-Aminosäurealkylester mit einem Isocyanatocarbonsäure-tert-butylester zum Harnstoff umgesetzt und dieser mit Natriumhydrid cyclisiert (Schritte A und B). Es kann auch von einem in der 4-Position durch die Gruppen R¹³ und R1-A- substituierten Hydantoin ausgegangen werden und dieses mit einem Bromcarbonsäure-tert-butylester alkyliert werden (Schritt C). Das Zwischenprodukt der Formel VIa kann in situ in die folgende Synthesestufe eingesetzt werden oder isoliert werden.

Gemäß einer der durchgeführten Varianten wurde das Zwischenprodukt der Formel VIa mit einer Verbindung der Formel R³⁰-LG (siehe allgemeine Synthesebeschreibung), zum Beispiel mit 4-Chlormethylstilben der Formel C₆H₅-CH=CH-C₆H₄-(4-CH₂Cl), umgesetzt (Schritt D). Nachfolgend wurde mit Trifluoressigsäure die tert-Butylestergruppe zur Säure gespalten, wobei eine Verbindung der Formel IIa erhalten wurde (Schritt E).

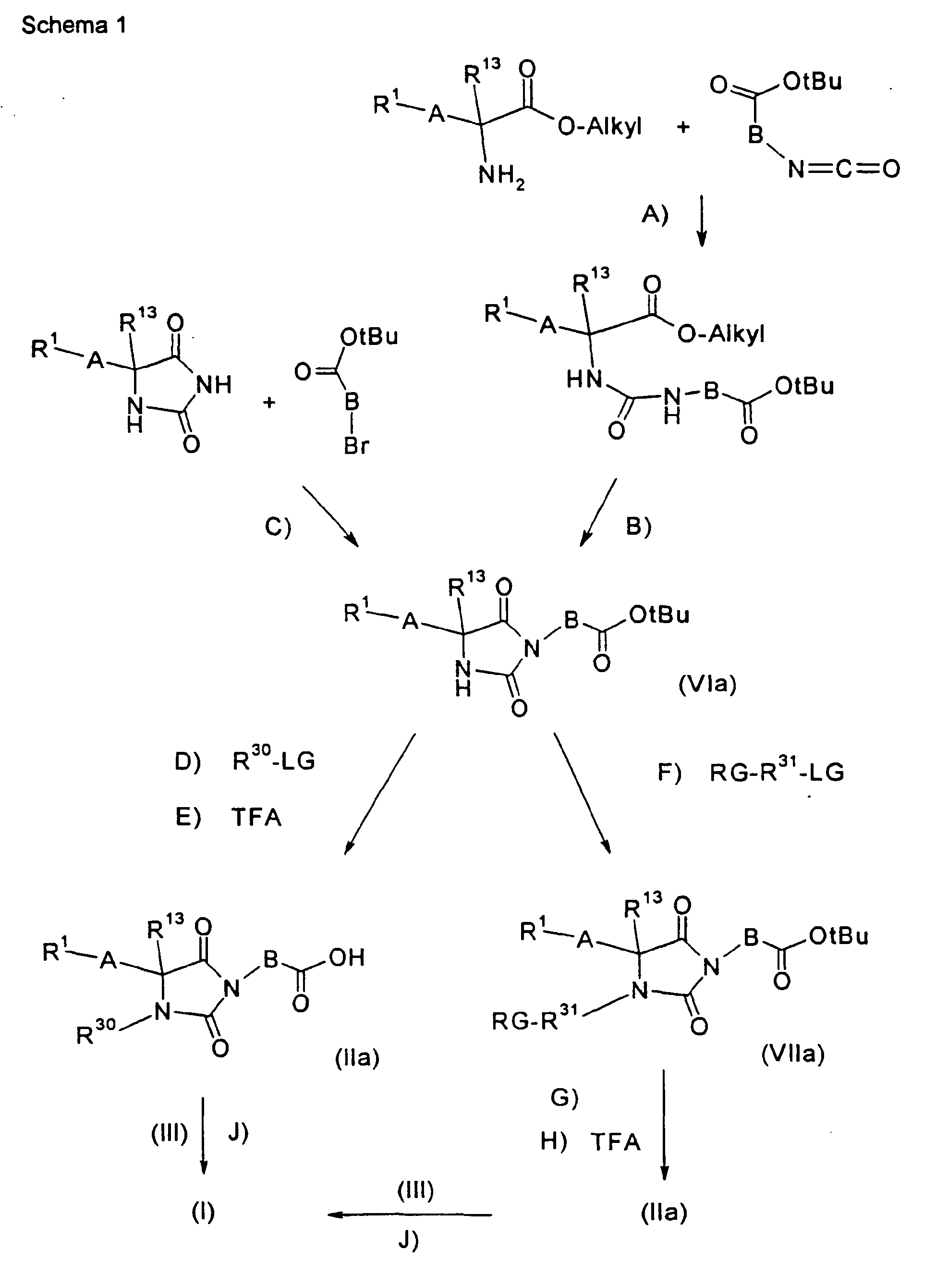

Gemäß einer anderen Variante wurde die Verbindung der Formel VIa mit einer Verbindung der Formel RG-R³¹-LG (siehe allgemeine Synthesebeschreibung) zu einer Verbindung der Formel VIIa umgesetzt (Schritt F), in der dann die Gruppe RG-R³¹- in die Gruppe R³⁰ umgewandelt wurde (allgemeiner Schritt G). Im Schritt G durchgeführte Umwandlungen sind im einzelnen in den Schemata 2 und 3 dargestellt.

Schema 2 zeigt die Umwandlung eines cyan-substituierten Hydantoins der Formel VIIb, d. h. einer Verbindung der Formel VIIa mit RG = Cyan, durch Reduktion der Cyangruppe zur Aldehydgruppe (Schritt K) und Umsetzung der Aldehydgruppe in einer Horner-Emmons-Reaktion zur Verbindung der Formel VIIc (Schritt L). Verbindungen der Formel VIIb wurden durch Alkylierung von Verbindungen der Formel VIa beispielsweise mit 4-Cyanbenzylbromid erhalten.

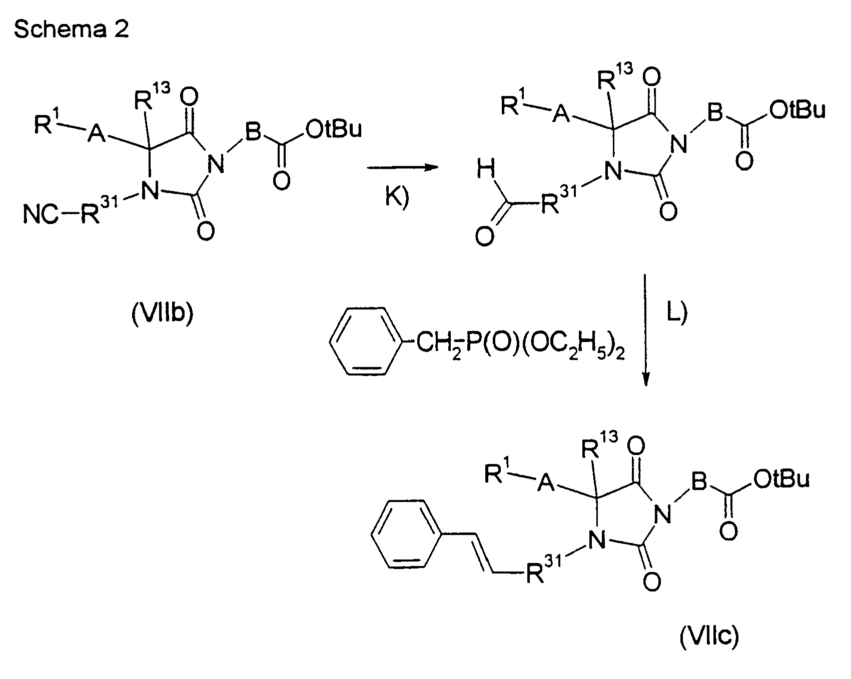

Schema 3 zeigt die Umwandlung eines halogen-substituierten Hydantoins der Formel VIId, d. h. einer Verbindung der Formel VIIa mit RG = Halogen (in der Formel VIId steht Hal für Halogen, insbesondere für lod oder Brom). Verbindungen der Formel VIId wurden durch Alkylierung von Verbindungen der Formel VIa beispielsweise mit 4-lodbenzylbromid zu 3-(4-lodbenzyl)-hydantoinen erhalten und wurden in einer Heck-Reaktion in Gegenwart eines Palladiumkatalysators mit Styrolen zu Verbindungen der Formel VIIe (Schritt M) oder mit Phenylacetylenen zu Verbindungen der Formel VIIf (Schritt N) umgesetzt (siehe R. F. Heck, Org. React. 1982, 27, S. 345). 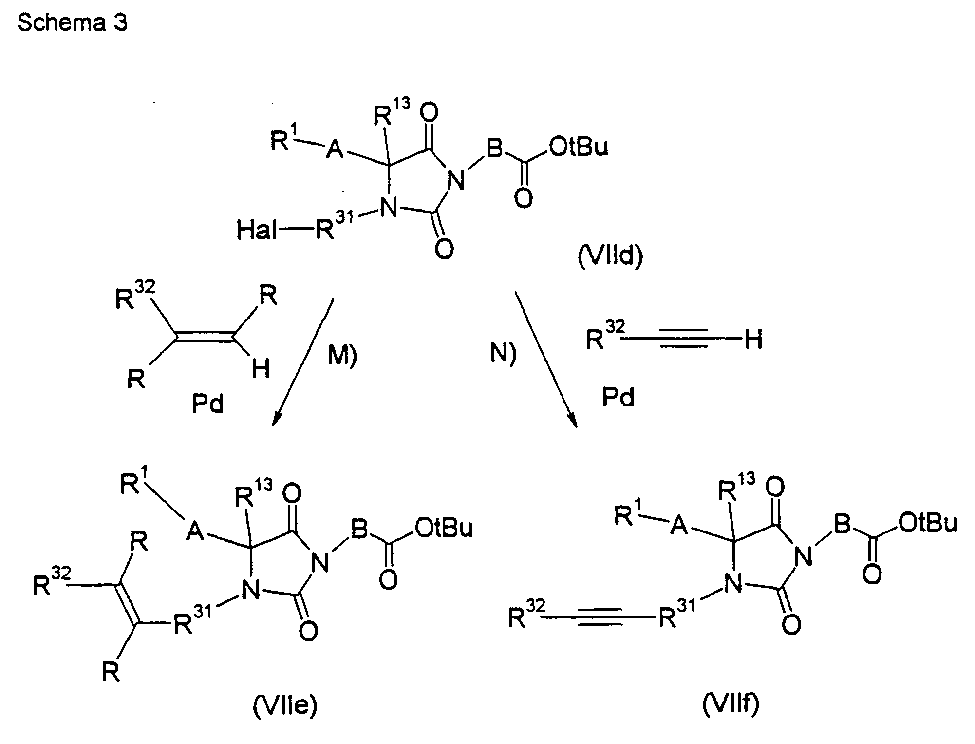 In den in den Schritten L, M, N bzw. allgemein im Schritt G erhaltenen Verbindungen wurde dann wiederum die tert-Butylestergruppe mit Trifluoressigsäure in die Carbonsäuregruppe überführt (Schritt H). Das in den Schritten E oder H erhaltene Zwischenprodukt der Formel IIa wurde dann mit einer Aminoverbindung der Formel Formel III gekuppelt, in der eine gegebenenfalls vorhandene Carbonsäuregruppe als tert-Butylester geschützt war, und schließlich nach Abspaltung der tert-Butylester-Schutzgruppe die Zielverbindung der Formel I erhalten (Schritt J; Schema 1). Die in die einzelnen Schritte eingesetzten Ausgangsverbindungen ergeben sich aus den Strukturen der einzelnen Beispiele.

In den einzelnen Syntheseschritten wurde nach den folgenden allgemeinen Verfahrenvorschriften gearbeitet.

### Schritte A und B

Der α-Aminosäurealkylester (90 mmol) wurde in 200 ml DMF gelöst und mit 1 Equivalent des lsocyanatocarbonsäure-tert-butylesters versetzt. Das Gemisch wurde 12 h bei Raumtemperatur gerührt (vollständiger Umsatz nach DC-Kontrolle). Die Lösung des entstandenen Harnstoffes in DMF (Gesamtvolumen 230 ml) wurde ohne weitere Reinigung und Aufarbeitung in die folgende Reaktion eingesetzt.

Zur Cyclisierung des Harnstoffs zum Hydantoin wurde ein Aliquot der Harnstofflösung auf 0 °C gekühlt und mit 1,2 Equivalenten (bezogen auf den Harnstoff) Natriumhydrid (als 55 %ige Suspension in Mineralöl) versetzt. Das Gemisch wurde 15 min bei 0 °C und anschließend 2 h bei Raumtemperatur gerührt. (vollständiger Umsatz nach DC-Kontrolle (Heptan/MTBE, 1/1)). Das Lösungsmittel wurde am Rotationsverdampfer entfernt. Der Rückstand wurde durch Flash-Chromatographie gereinigt (Kieselgel, Heptan/MTBE, 6/4). Man erhielt das cyclisierte Hydantoin in einer Ausbeute von > 90%.

### Schritt C

Zur Herstellung der Verbindungen der Formel Vla kann das Ausgangs-Hydantoin in DMF gelöst werden und mit 1,2 Equivalenten Natriumhydrid (55 %ige Suspension in Mineralöl) versetzt werden. Das Gemisch wird dann im allgemeinen 4 h bei Raumtemperatur gerührt. Nach Zugabe von 1,7 Equivalenten des Bromcarbonsäure-tert-butylesters wird bei Raumtemperatur über Nacht weitergerührt. Das Lösungsmittel wird am Rotationsverdampfer entfernt. Der Rückstand wird durch Flash-Chromatographie gereinigt.

### Schritt D (Umsetzung mit 4-Chlormethylstilben)

Zu dem im Schritt B erhaltenen Hydantoin wurden je 1,1 Equivalente (bezogen auf das Hydantoin) 4-Chlormethylstilben und Natriumhydrid zugegeben und das Gemisch 4 h bei Raumtemperatur gerührt. Die Reaktion wurde durch Zugabe von Wasser gequencht und das Lösungsmittel am Rotationsverdampfer abgezogen. Der ölige Rückstand wurde in Essigester aufgenommen und mit Wasser gewaschen. Die organische Phase wurde über Magnesiumsulfat getrocknet und das Lösungsmittel im Vakuum entfernt. Der Rückstand wurde durch Flash-Chromatographie gereinigt (Kieselgel, Hexan/MTBE, 6/4). Neben einer Fraktion, die das reine 3-alkylierte Hydantoinderivat in einer Ausbeute von 50 - 60 % enthielt, wurden weitere Fraktionen erhalten, die das Produkt in leicht verunreinigter Form enthielten.

### Schritt F (Umsetzung mit 4-Cyanbenzylbromid)

Das in Schritt B erhaltene alkylierte Hydantoin (14 mmol) wurde in 50 ml DMF gelöst und mit 1,2 Equivalenten Caesiumcarbonat und 1 Equivalent 4-Cyanbenzylbromid versetzt und die Mischung 16 - 20 h bei Raumtemperatur gerührt. Das Reaktionsgemisch wurde durch Zugabe von Wasser gequencht und das Lösungsmittel am Rotationsverdampfer abgezogen. Der ölige Rückstand wurde in Essigester aufgenommen und mit Wasser gewaschen. Die organische Phase wurde über Magnesiumsulfat getrocknet und das Lösungsmittel im Vakuum entfernt. Man erhielt das 3-(4-Cyanobenzyl)-hydantoinderivat in einer Ausbeute von ca. 60 %.

### Schritt K

Das im Schritt F erhaltene 3-(4-Cyanobenzyl)-hydantoinderivat (ca. 14 mmol) wurde in 360 ml einer Mischung von Pyridin/Essigsäure/Wasser (2/1/1) gelöst, auf 0 °C gekühlt und mit 25,1 g Natriumhypophosphit (Monohydrat) und 4,17 g feuchtem Raney-Nickel versetzt. Nach 6 h Rühren bei 60 °C wurden die Lösungsmittel im Vakuum entfernt und der Rückstand in Essigester aufgenommen. Die Lösung wurde mit 10%iger Citronensäurelösung, gesättigter Natriumbicarbonatlösung und gesättigter Natriumchloridlösung gewaschen und über Magnesiumsulfat getrocknet. Nach Filtration und Einengen wurde das erhaltene 3-(4-Formylbenzyl)-hydantoinderivat ohne weitere Reinigung in den nächsten Schritt eingesetzt.

### Schritt L

Zu 1,16 g (4,8 mmol) Diethylbenzylphosphonat (in 20 ml DMF gelöst) wurden 150 mg (6,24 mmol) Natriumhydrid (60 %ige Suspension in Öl) gegeben. Die Mischung wurde 15 Minuten bei Raumtemperatur gerührt. Anschließend wurden 2,0 g (4,8 mmol) des in Schritt K erhaltenen 3-(4-Formylbenzyl)-hydantoinderivats zugegeben und die Mischung 16 h bei Raumtemperatur gerührt. Das Lösungsmittel wurde unter vermindertem Druck abgezogen und der Rückstand in Essigester aufgenommen. Die Lösung wurde mit Wasser gewaschen, über Magnesiumsulfat getrocknet und filtriert. Das Filtrat wurde im Vakuum eingeengt und der ölige Rückstand über Kieselgel chromatographiert (n-Heptan/Essigester, 6/1). Die die Zielverbindung enthaltenden Fraktionen wurden vereinigt und vom Lösungsmittel befreit. Man erhielt ca. 60% des gewünschten Stilbens.

### Schritt F (Umsetzung mit 4-lodbenzylbromid)

Das in Schritt B erhaltene alkylierte Hydantoin (14 mmol) wurde in 50 ml DMF gelöst, mit je 1,1 Equivalenten Phosphazen P-1 als Base und 4-lodbenzylbromid versetzt und die Mischung 2 - 3 h bei Raumtemperatur gerührt. Das Reaktionsgemisch wurde durch Zugabe von Wasser gequencht und das Lösungsmittel am Rotationsverdampfer abgezogen. Der ölige Rückstand wurde in Essigester aufgenommen und mit Wasser gewaschen. Die organische Phase wurde über Magnesiumsulfat getrocknet und das Lösungsmittel im Vakuum entfernt. Der Rückstand wurde durch Flash-Chromatographie gereinigt (Kieselgel, Hexan/MTBE, 6/4). Man erhielt das reine 3-(4-lodbenzyl)-hydantoinderivat in einer Ausbeute von ca. 60 %.

### Schritt M

Das in Schritt F erhaltene 3-(4-lodbenzyl)-hydantoinderivat (7 mmol) wurde in 30 ml DMF gelöst und mit 2 Equivalenten des Styrols der Formel R³²-CR=CHR und Palladium(II)aceta/Triphenylphosphin versetzt. Das Gemisch wurde 14 h auf 90 °C erhitzt (vollständiger Umsatz nach DC-Kontrolle (Ethylacetat/Petrolether, 1/4)). Das Lösungsmittel wurde im Vakuum entfernt und der Rückstand durch Flash-Chromatographie gereinigt (Kieselgel, Ethylacetat/Petrolether, 1/4). Man erhielt das Produkt in einer Ausbeute von ca. 80 %.

### Schritt N

Das 3-(4-lodobenzyl)-hydantoinderivat (7 mmol) wurde in 30 ml DMF gelöst und mit 2 Equivalenten des Phenyl acetylens der Formel R³²-C≡CH und Palladium(II)acetat/Triphenylphosphin versetzt. Das Gemisch wurde 14 h auf 90 °C erhitzt (vollständiger Umsatz nach DC-Kontrolle (Ethylacetat/Petrolether, 1/4). Das Lösungsmittel wurde im Vakuum entfernt und der Rückstand durch Flash-Chromatographie gereinigt (Kieselgel, Ethylacetat/Petrolether, 1/4). Man erhielt das Produkt in einer Ausbeute von ca. 80 %.

### Schritte E und H

Der in den Schritten D, L, M oder N erhaltene tert-Butylester wurde zur Überführung in die Carbonsäure 1 h bei Raumtemperatur in Trifluoressigsäure/Dichlormethan (1/1; ca. 20 ml/mmol) geschüttelt. Die Trifluoressigsäure wurde am Rotationsverdampfer entfernt und der Rückstand gefriergetrocknet. Man erhielt die Carbonsäure in quantitativer Ausbeute.

### Schritt J

Die in Schritt E oder H erhaltene Carbonsäure (ca. 2 mmol) wurde in 10 ml DMF gelöst und mit 1 Equivalent der zu kuppelnden Aminoverbindung, in der eine Carbonsäuregruppe als tert-Butylester vorlag, und 1 Equivalent HOBt versetzt. Das Gemisch wurde auf 0 °C gekühlt, mit 1 Equivalent DCC versetzt und 1 h bei 0 °C gerührt. Anschließend wurde 4 h bei Raumtemperatur gerührt (vollständiger Umsatz nach DC-Kontrolle (Dichlormethan/Methanol, 20/1)). Das Gemisch wurde filtriert und das Lösungsmittel im Vakuum entfernt. Reinigung des Rückstandes durch Flash-Chromatographie (Kieselgel, Dichlormethan/Methanol, 20/1) ergab das Kupplungsprodukt in einer Ausbeute von > 80 %. Zur Spaltung der tert-Butylester-Schutzgruppe wurde das Kupplungsprodukt in Trifluoressigsäure/Dichlormethan (1/1; ca. 20 ml/mmol) gelöst und 1 h bei Raumtemperatur geschüttelt. Das Lösungsmittel wurde am Rotationsverdampfer entfernt. Der Rückstand wurde, teilweise nach Zusatz von Essigsäure/Wasser, gefriergetrocknet oder durch Chromatographie gereinigt. Man erhielt die unten in den einzelnen Beispielen bezeichnete Säure in quantitativer Ausbeute.

Anstatt mit Aminoverbindungen in Lösung können die in Schritt E oder H erhaltenen Carbonsäuren auch mit harzgebundenen Aminoverbindungen gekuppelt werden, die auch am Harz aufgebaut werden können. Soll als Aminoverbindung eine Aminocarbonsäure in die Kupplung eingesetzt werden, wird zur Anknüpfung der Aminocarbonsäure oder des C-terminalen Bausteins einer Aminoverbindung, die am Harz aufgebaut werden soll, an den polymeren Träger das Harz (Wang, Polystyrol, Bachem) mit 2 Equivalenten der Fmoc-geschützten Aminocarbonsäure, 2 Equivalenten HOBt und 2 Equivalenten TOTU versetzt. Zu dem Gemisch werden 2 Equivalente Diisopropylethylamin, gelöst in DMF (10 ml/g Träger), gegeben. Das Gemisch wird 12 h bei 40 °C geschüttelt. Anschließend wird zu dem Gemisch 1 Equivalent Acetanhydrid und 1 Equivalent Diisopropylethylamin gegeben und weitere 30 min bei Raumtemperatur geschüttelt. Das Lösungsmittel wird durch Filtration entfernt, der Rückstand wird jeweils 5 mal mit DMF, Toluol und Dichlormethan gewaschen. Die Beladung des Harzes wird an einer Probe durch Fmoc-Abspaltung nach Standardmethoden der Peptidsynthese bestimmt (die Beladung beträgt je nach eingesetzter Aminosäure im allgemeinen 0,3 bis 0,6 mmol/g Harz). Zur Abspaltung der Fmoc-Gruppe wird das Harz dann in einer 20 %igen Lösung von Piperidin in DMF suspendiert (10 ml Lösung/g Harz) und für 20 min geschüttelt. Die Lösung wird abfiltriert und der Vorgang wird wiederholt. Danach wird das Harz mehrfach mit DMF und Dichlormethan gewaschen. Die harzgebundene Aminosäure (100 mg Harz) wird mit 2 Equivalenten der zu kuppelnden Carbonsäure, 2 Equivalenten HOBt und 2 Equivalenten TOTU versetzt. Zu dem Gemisch werden 2 Equivalente Diisopropylethylamin, gelöst in 2 ml DMF, gegeben. Das Gemisch wird 12 h bei Raumtemperatur geschüttelt. Es wird filtriert und danach jeweils 5 mal mit DMF, Toluol und Dichlormethan gewaschen. Zur Abspaltung des Kupplungsprodukts vom Träger wird das gereinigte Harz mit 1 ml Trifluoressigsäure/Dichlormethan (1/1) versetzt und 1 h geschüttelt. Die Abspaltungslösung wird eingeengt, der Rückstand zur Reinigung mit Essigester über eine mit Kieselgel gefüllte Kartusche filtriert und das Lösungsmittel entfernt. Wurden Verbindungen nach dieser Vorgehensweise hergestellt, so ist unten angegeben, daß an Harz gebundene Aminoverbindungen eingesetzt wurden.

Allgemeines Verfahren zur Herstellung von im Schritt J eingesetzten 3-substituierten 3-Amino-propionsäure-tert-butylestern

Die entsprechende 3-substituierte Acrylsäure (0,1 mol) wurde mit 1,1 Equivalenten Oxalylchlorid in 100 ml Dichlormethan gelöst. Das Gemisch wurde 4 h bei Raumtemperatur gerührt. Das Lösungsmittel wurde am Rotationsverdampfer entfernt. Der Rückstand wurde in 100 ml tert-Butanol aufgenommen und 2 h bei Raumtemperatur gerührt. Nach beendeter Reaktion wurde das Lösungsmittel am Rotationsverdampfer entfernt. Der Rückstand wurde in Diethylether gelöst und mit Wasser, Natriumhydrogencarbonatlösung und erneut mit Wasser gewaschen. Die organische Phase wurde über Magnesiumsulfat getrocknet und das Lösungsmittel im Vakuum entfernt. Man erhielt den 3-substituierten Acrylsäure-tert-butylester in einer Ausbeute von > 80 %.

Zur Einführung der Aminogruppe wurden zu einer Lösung von (R)-(+)-N-Benzyl-N-(1-phenyl-ethyl)-amin (60 mmol) in 100 ml THF bei -70 °C über den Zeitraum von 1 h 0,95 Equivalente n-Butyllithium (in n-Hexan) zugetropft. Das Gemisch wurde 1 h bei dieser Temperatur gerührt, dann wurde eine Lösung des 3-substituierten Acrylsäure-tert-butylesters (0,9 Equivalente) in 75 ml THF über den Zeitraum von 1 h zugetropft. Das Gemisch wurde 2 h bei -70 °C gerührt. Nach Entfernen der Kühlung wurden 115 ml 5 %ige Citronensäurelösung zugetropft. Die Lösung wurde 1 h gerührt, mit Essigester versetzt und mit Wasser gewaschen. Die organische Phase wurde mit Natriumhydrogencarbonatlösung und Wasser gewaschen und über Magnesiumsulfat getrocknet. Das Lösungsmitel wurde im Vakuum entfernt. Der Rückstand wurde durch Flash-Chromatographie gereinigt (Kieselgel, Hepta/Essigester, 9/1). Man erhielt den 3-substituierten 3-(N-Benzyl-N-(1-phenylethyl)-amino)-propionsäure-tert-butylester in einer Ausbeute von ca. 50 % als gelbes 01. Zur Abspaltung der Benzylgruppe und der Phenylethylgruppe wurde die Substanz (ca. 30 mmol) in 200 ml eines Gemisches aus Essigester/Essigsäure (4/1) gelöst und mit 1,5 g Pd(OH)₂ versetzt. Unter einer Wasserstoffatmosphäre wurde 8 h bei Raumtemperatur hydriert. Der Katalysator wurde abfiltriert und das Filtrat am Rotationsverdampfer eingeengt. Der Rückstand wurde in Ether/Wasser aufgenommen. Die wäßrige Phase wurde mit Natriumhydrogencarbonat neutralisiert und mehrfach mit Ether extrahiert. Die vereinigten organischen Phasen wurden über Magnesiumsulfat getrocknet und vorsichtig am Rotationsverdampfer eingeengt. Man erhielt den 3-substituierten 3-Amino-propionsäure-tert-butylester als dünnflüssiges, leichtflüchtiges Öl in einer Ausbeute von > 50 %.

### Beispiel 1

### (R)-3-((S)-2-(4,4-Dimethyl-3-(4-styryl-benzyl)-2,5-dioxoimidazolidin-1-yl)-2-(2-methylpropyl)-acetylamino)-3-methyl-propionsäure

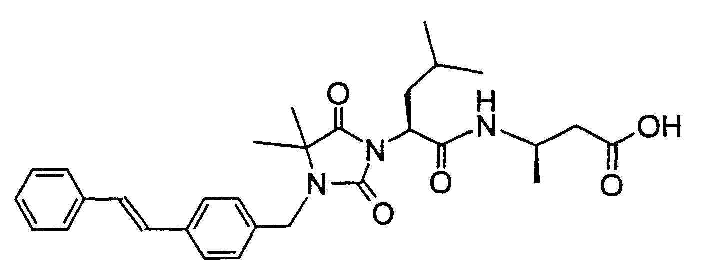

Die Verbindung wurde nach den allgemeinen Herstellungsverfahren, Schritte A, B, D, E und J, hergestellt. Im Schritt D wurde (S)-2-(4,4-Dimethyl-2,5-dioxoimidazolidin-1-yl)-2-(2-methylpropyl)-essigsäure-tert-butylester mit 4-Chlormethylstilben alkyliert. Im Schritt J wurde als Aminoverbindung (R)-3-Amino-3-methyl-propionsäure-tert-butylester eingesetzt.
Ausbeute: 170 mg
ES(+)-MS: 520,4 (M+H)

### Beispiel 3

### (R)-3-((S)-2-(4,4-Dimethyl-3-(4-styryl-benzyl)-2,5-dioxoimidazolidin-1-yl)-2-(2-methylpropyl)-acetylamino)-3-phenyl-propionsäure

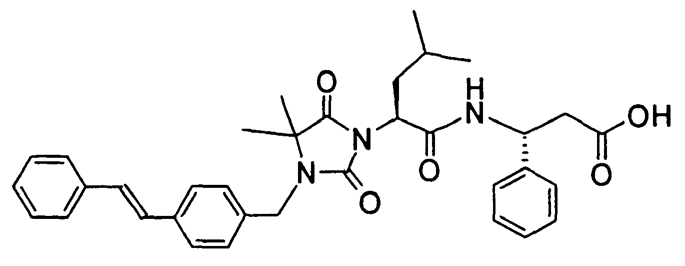

Die Verbindung wurde nach den allgemeinen Herstellungsverfahren, Schritte A, B, D, E und J, hergestellt. Im Schritt J wurde als Aminoverbindung (R)-3-Amino-3-phenyt-propionsäure-tert-butylester eingesetzt.
Ausbeute: 130 mg
ES(+)-MS: 582,6 (M+H)

### Beispiel 4

### 3-((S)-2-(4,4-Dimethyl-3-(4-styryl-benzyl)-2,5-dioxoimidazolidin-1-yl)-2-(2-methylpropyl)-acetylamino)-propionsäure

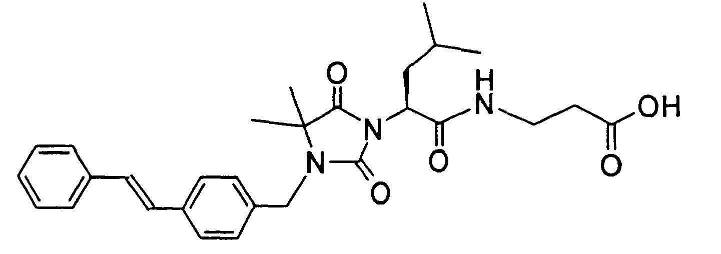

Die Verbindung wurde nach den allgemeinen Herstellungsverfahren, Schritte A, B, D, E und J, hergestellt. Im Schritt J wurde als Aminoverbindung β-Alanin-tert-butylester eingesetzt.
Ausbeute: 102 mg
ES(+)-MS: 506,5 (M+H)

### Beispiel 5

### (R)-3-((S)-2-(4,4-Dimethyl-3-(4-styryl-benzyl)-2,5-dioxoimidazolidin-1-yl)-2-(2-methylpropyl)-acetylamino)-3-(3,4-methylendioxyphenyl)-propionsäure

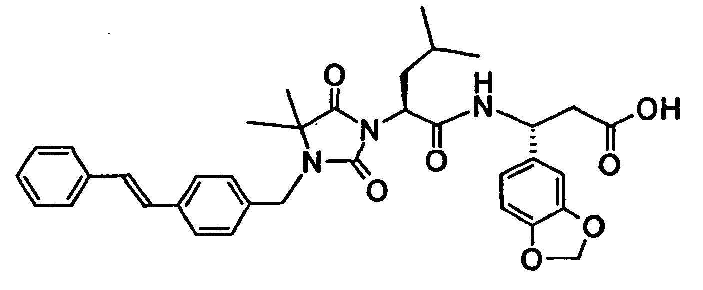

Die Verbindung wurde nach den allgemeinen Herstellungsverfahren, Schritte A, B, D, E und J, hergestellt. Im Schritt J wurde als Aminoverbindung (R)-3-Amino-3-(3,4-methylendioxyphenyl)-propionsäure-tert-butylester eingesetzt.
Ausbeute: 320 mg
ES(+)-MS: 626,2 (M+H)

### Beispiel 8

### (R)-3-((S)-2-(4,4-Dimethyl-3-(4-(2-(2-methylphenyl)-vinyl)-benzyl)-2,5-dioxoimidazolidin-1-yl)-2-(2-methylpropyl)-acetylamino)-3-methyl-propionsäure

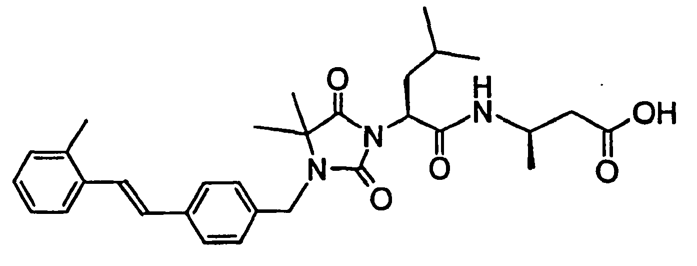

Die Verbindung wurde nach den allgemeinen Herstellungsverfahren, Schritte A, B, F (Umsetzung mit 4-Cyanbenzylbromid), K, L, H und J hergestellt. Im Schritt J wurde als Aminoverbindung (R)-3-Amino-3-methyl-propionsäure-tert-butylester eingesetzt.
Ausbeute: 260 mg
ES(+)-MS: 534,4 (M+H)

### Beispiel 9

### (R)-3-((S)-2-(4,4-Dimethyl-3-(4-(2-(2-methylphenyl)-vinyl)-benzyl)-2,5-dioxoimidazolidin-1-yl)-2-(2-methylpropyl)-acetylamino)-3-phenyl-propionsäure

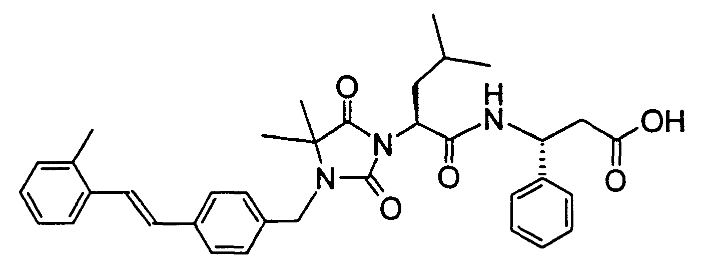

Die Verbindung wurde nach den allgemeinen Herstellungsverfahren, Schritte A, B, F (Umsetzung mit 4-Cyanbenzylbromid), K, L, H und J hergestellt. Im Schritt J wurde als Aminoverbindung (R)-3-Amino-3-phenyl-propionsäure-tert-butylester eingesetzt.
Ausbeute: 85 mg
ES(+)-MS: 596,4 (M+H)

### Beispiel 10

### 3-((S)-2-(4,4-Dimethyl-3-(4-styryl-benzyl)-2,5-dioxoimidazolidin-1-yl)-2-(2-methylpropyl)-acetylamino)-3-(3-methoxyphenyl)-propionsäure

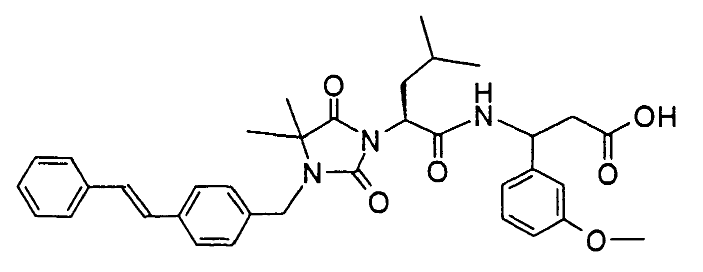

Die Verbindung wurde nach den allgemeinen Herstellungsverfahren, Schritte A, B, D, E und J, hergestellt. Im Schritt J wurde als Aminoverbindung an Wang-Harz gebunden 3-Amino-3-(3-methoxyphenyl)-propionsäure eingesetzt.
Ausbeute: 2,9 mg
ES(+)-MS: 612,7 (M+H)

### Beispiel 11

### 3-((S)-2-(4,4-Dimethyl-3-(4-styryl-benzyl)-2,5-dioxoimidazolidin-1-yl)-2-(2-methytpropyl)-acetylamino)-3-(4-fluorphenyl)-propionsäure

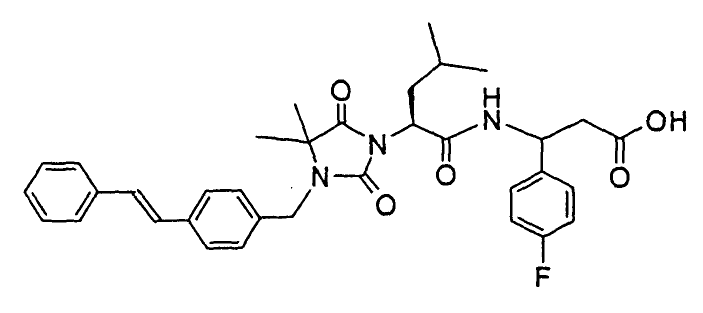

Die Verbindung wurde nach den allgemeinen Herstellungsverfahren, Schritte A, B, D, E und J, hergestellt. Im Schritt J wurde als Aminoverbindung an Wang-Harz gebundene 3-Amino-3-(4-fluorphenyl)-propionsäure eingesetzt.
Ausbeute: 4,1 mg
ES(+)-MS: 600,7 (M+H)

### Beispiel 12

### 3-((S)-2-(4,4-Dimethyl-3-(4-styryl-benzyl)-2,5-dioxoimidazolidin-1-yl)-2-(2-methylpropyl)-acetylamino)-3-(3-fluor-4-methoxyphenyl)-propionsäure

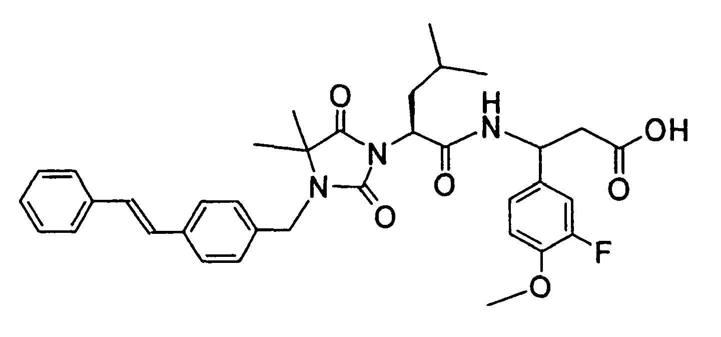

Die Verbindung wurde nach den allgemeinen Herstellungsverfahren, Schritte A, B, D, E und J, hergestellt. Im Schritt J wurde als Aminoverbindung an Wang-Harz gebundene 3-Amino-3-(3-fluor-4-methoxyphenyl)-propionsäure eingesetzt.
Ausbeute: 2,3 mg
ES(+)-MS: 630,7 (M+H)

### Beispiel 13

### 3-((S)-2-(4,4-Dimethyl-3-(4-styryl-benzyl)-2,5-dioxoimidazolidin-1-yl)-2-(2-methylpropyl)-acetylamino)-3-(3-methylphenyl)-propionsäure

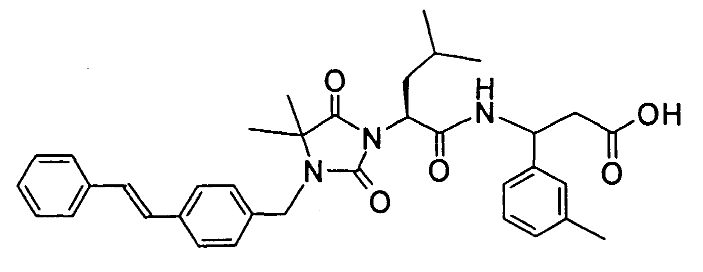

Die Verbindung wurde nach den allgemeinen Herstellungsverfahren, Schritte A, B, D, E und J, hergestellt. Im Schritt J wurde als Aminoverbindung an Wang-Harz gebundene 3-Amino-3-(3-methylphenyl)-propionsäure eingesetzt.
Ausbeute: 5,0 mg
ES(+)-MS: 596,7 (M+H)

### Beispiel 14

### 3-((S)-2-(4,4-Dimethyl-3-(4-styryl-benzyl)-2,5-dioxoimidazolidin-1-yl)-2-(2-methylpropyl)-acetylamino)-3-(2-fluorphenyl)-propionsäure

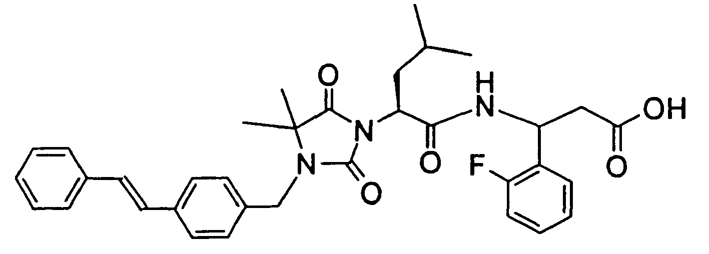

Die Verbindung wurde nach den allgemeinen Herstellungsverfahren, Schritte A, B, D, E und J, hergestellt. Im Schritt J wurde als Aminoverbindung an Wang-Harz gebundene 3-Amino-3-(2-fluorphenyl)-propionsäure eingesetzt.
Ausbeute: 4,9 mg
ES(+)-MS: 600,7 (M+H)

### Beispiel 15

### 3-((S)-2-(4,4-Dimethyl-3-(4-styryl-benzyl)-2,5-dioxoimidazolidin-1-yl)-2-(2-methylpropyl)-acetylamino)-3-(3-fluorphenyl)-propionsäure

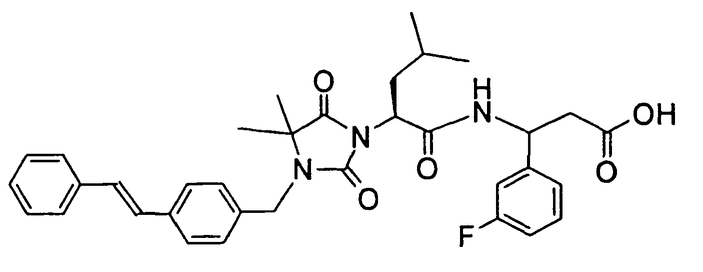

Die Verbindung wurde nach den allgemeinen Herstellungsverfahren, Schritte A, B, D, E und J, hergestellt. Im Schritt J wurde als Aminoverbindung an Wang-Harz gebundene 3-Amino-(3-fluorphenyl)-propionsäure eingesetzt.
Ausbeute: 4,2 mg
ES(+)-MS: 600,7 (M+H)

### Beispiel 16

### 3-(4-Butylphenyl)-3-((S)-2-(4,4-dimethyl-3-(4-styryl-benzyl)-2,5-dioxoimidazolidin-1-yl)-2-(2-methylpropyl)-acetylamino)-propionsäure

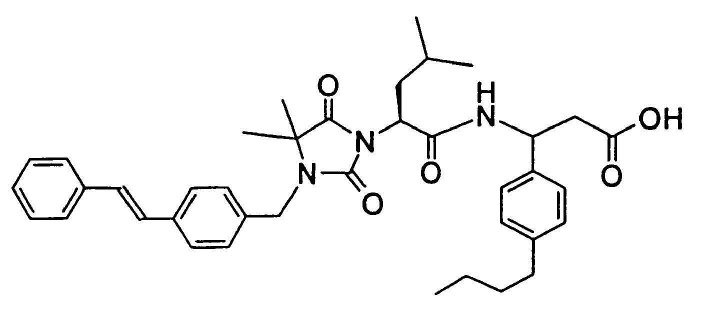

Die Verbindung wurde nach den allgemeinen Herstellungsverfahren, Schritte A, B, D, E und J, hergestellt. Im Schritt J wurde als Aminoverbindung an Wang-Harz gebundene 3-Amino-3-(4-butylphenyl)-propionsäure eingesetzt.
Ausbeute: 8,5 mg
ES(+)-MS: 638,7 (M+H)

### Beispiel 17

### 3-(4-Chlorphenyl)-3-((S)-2-(4,4-Dimethyl-3-(4-styryl-benzyl)-2,5-dioxoimidazolidin-1-yl)-2-(2-methylpropyl)-acetylamino)-propionsäure

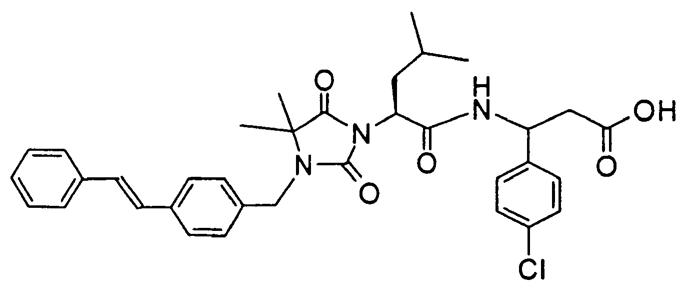

Die Verbindung wurde nach den allgemeinen Herstellungsverfahren, Schritte A, B, D, E und J, hergestellt. Im Schritt J wurde als Aminoverbindung an Wang-Harz gebundene 3-Amino-3-(4-chlorphenyl)-propionsäure eingesetzt.
Ausbeute: 7,9 mg
ES(+)-MS: 617,7 (M+H)

### Beispiel 18

### (R)-3-((S)-2-(4,4-Dimethyl-3-(4-(2-phenyl-ethinyl)-benzyl)-2,5-dioxoimidazolidin-1-yl)-2-(2-methylpropyl)-acetylamino)-3-methyl-propionsäure

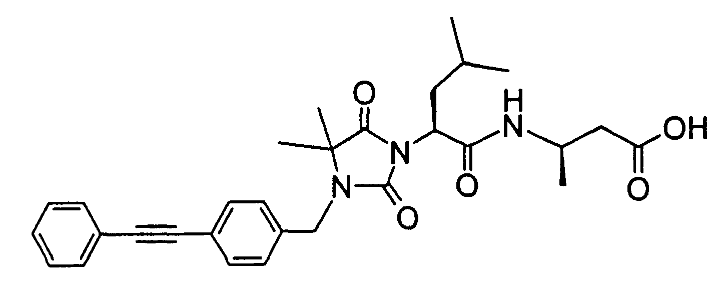

Die Verbindung wurde nach den allgemeinen Herstellungsverfahren, Schritte A, B, F (Umsetzung mit 4-lodbenzylbromid), N (Umsetzung mit Phenylacetylen), H und J, hergestellt. Im Schritt J wurde als Aminoverbindung (R)-3-Amino-3-methyl-propionsäure-tert-butylester eingesetzt.
Ausbeute: 15 mg

### Beispiel 19

### (R)-3-((S)-2-(4,4-Dimethyl-3-(4-(2-(2-fluorphenyl)-ethinyl)-benzyl)-2,5-dioxoimidazolidin-1-yl)-2-(2-methylpropyl)-acetylamino)-3-methyl-propionsäure

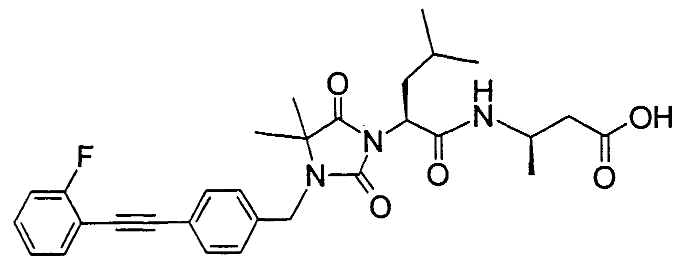

Die Verbindung wurde nach den allgemeinen Herstellungsverfahren, Schritte A, B, F (Umsetzung mit 4-lodbenzylbromid), N (Umsetzung mit 2-Fluorphenyl-acetylen), H und J, hergestellt. Im Schritt J wurde als Aminoverbindung (R)-3-Amino-3-methyl-propionsäure-tert-butylester eingesetzt.
Ausbeute: 20 mg

### Untersuchung der biologischen Aktivität

Als Testmethode für die Wirksamkeit der Verbindungen der Formel I auf die Interaktion zwischen VCAM-1 und VLA-4 wird ein Assay verwendet, der für diese Interaktion spezifisch ist. Die zellulären Bindungspartner, d. h. die VLA-4-Integrine, werden in ihrer natürlichen Form als Oberflächenmoleküle auf humanen U937-Zellen (ATCC CRL 1593), die zur Gruppe der Leukozyten gehören, angeboten. Als spezifische Bindungspartner werden gentechnisch hergestellte rekombinante lösliche Fusionsproteine, bestehend aus der extrazytoplasmatischen Domäne von humanen VCAM-1 und der konstanten Region eines humanen Immunglobulins der Subklasse IgG1, verwendet.

### Testmethode

Assay zur Messung der Adhäsion von U937-Zellen (ATCC CRL 1593) an hVCAM-1 (1-3)-IgG

### 1. Herstellung von humanem VCAM-1(1-3)-IgG und humanem CD4-IgG

Eingesetzt wurde ein genetisches Konstrukt zur Expression der extrazellulären Domäne des humanen VCAM-1, verbunden mit der genetischen Sequenz der schweren Kette des humanen lmmunglobulins IgG1 (Hinge, CH2 und CH3 Regionen) (von Dr. Brian Seed, Massachusetts General Hospital, Boston, USA; vgl. Damle und Aruffo, Proc. Natl. Acad. Sci. USA 1991, 88, 6403-6407). Das lösliche Fusionsprotein hVCAM-1(1-3)-IgG enthielt die drei aminoterminalen extrazellulären Immunglobulin-ähnlichen Domänen des humanen VCAM-1 (Damle und Aruffo, Proc. Natl. Acad. Sci. USA 1991, 88, 6403-6407). CD4-IgG (Zettlmeissl et al., DNA and Cell Biology 1990, 9, 347) diente als Fusionsprotein für negative Kontrollen. Die rekombinanten Proteine wurden als lösliche Proteine nach DEAE/Dextranvermittelter DNA-Transfektion in COS-Zellen (ATCC CRL1651) gemäß Standardprozeduren exprimiert (Ausubel et al., Current protocols in molecular biology, John Wiley & Sons, Inc., 1994).

### 2. Assay zur Messung der Adhäsion von U937-Zellen an hVCAM-1 (1-3)-IgG

2.1 96 well-Mikrotitertestplatten (Nunc Maxisorb) wurden mit 100 µl/well einer Ziege-anti-human-IgG-Antikörperlösung (10 µg/ml in 50 mM Tris, pH 9,5) 1 Stunde bei Raumtemperatur inkubiert. Nach Entfernen der Antikörperlösung wurde einmal mit PBS gewaschen.
2.2 150 µl/well eines Blockierungspuffers (1 % BSA in PBS) wurde 0,5 Stunden bei Raumtemperatur auf den Platten inkubiert. Nach Entfernen des Blockierungspuffers wurde einmal mit PBS gewaschen.
2.3 100 µl pro well eines Zellkulturüberstandes von transfektierten COS-Zellen wurde für 1,5 Stunden bei Raumtemperatur auf den Platten inkubiert. Die COS-Zellen waren mit einem Plasmid transfiziert, welches für die drei N-terminalen Immunglobulin-ähnlichen Domänen des VCAM-1, gekoppelt an den Fc-Teil von humanem IgG₁ (hVCAM-1 (1-3)-IgG), codiert. Der Gehalt an hVCAM-1 (1-3)-IgG betrug ca. 0,5 - 1 µg/ml. Nach Entfernen des Kulturüberstandes wurde einmal mit PBS gewaschen.
2.4 Die Platten wurden mit 100 µl/well Fc-Rezeptor-Blockpuffer (1 mg/ml γ-Globulin, 100 mM NaCl, 100 µM MgCl₂, 100 µM MnCl₂, 100 µM CaCl₂, 1 mg/ml BSA in 50 mM HEPES, pH 7,5) für 20 Minuten bei Raumtemperatur inkubiert. Nach Entfernen des Fc-Rezeptor-Blockpuffers wurde einmal mit PBS gewaschen.
2.5 20 µl Bindungspuffer (100 mM NaCl, 100 µM MgCl₂, 100 µM MnCl₂, 100 µM CaCl₂, 1 mg/ml BSA in 50 mM HEPES, pH 7,5) wurden vorgelegt, die zu testenden Substanzen in 10 µl Bindungspuffer zugegeben und für 20 Minuten inkubiert. Als Kontrollen dienten Antikörper gegen VCAM-1 (BBT, Nr. BBA6) und gegen VLA-4 (Immunotech, Nr. 0764).
2.6 U937-Zellen wurden 20 Minuten in Fc-Rezeptor-Blockpuffer inkubiert und anschließend in einer Konzentration von 1 x 10⁶/ml und in einer Menge von 100 µl pro well zupipettiert (Endvolumen 125 µl/well).
2.7 Die Platten wurden in einem 45°-Winkel in Stoppuffer (100 mM NaCl, 100 µM MgCl₂, 100 µM MnCl₂, 100 µM CaCl₂ in 25 mM Tris, pH 7,5) langsam eingetaucht und ausgeschlagen. Der Vorgang wurde wiederholt.
2.8 Anschließend wurden 50 µl/well einer Färbelösung (16,7 µg/ml Hoechst Farbstoff 33258, 4 % Formaldehyd, 0,5 % Triton-X-100 in PBS) 15 Minuten auf den Platten inkubiert.
2.9 Die Platten wurden ausgeschlagen und in einem 45°-Winkel in Stop-Puffer (100 mM NaCl, 100 µM MgCl₂, 100 µM MnCl₂, 100 µM CaCl₂ in 25 mM Tris, pH 7,5) langsam eingetaucht. Der Vorgang wurde wiederholt. Anschließend wurden die Platten mit der enthaltenen Flüssigkeit (Stop-Puffer) in einem Cytofluorimeter (Millipore) gemessen (Sensitivität: 5, Filter: Anregungswellenlänge: 360 nm, Emissionswellenlänge: 460 nm).

Die Intensität des von den angefärbten U937-Zellen emittierten Lichts ist ein Maß für die Zahl der an der Platte verbliebenen, an das hVCAM-1 (1-3)-IgG adhärierten U937-Zellen und somit ein Maß für die Fähigkeit der zugesetzten Testsubstanz, diese Adhäsion zu hemmen. Aus der Hemmung der Adhäsion bei verschiedenen Konzentrationen der Testsubstanz wurde die Konzentration IC₅₀ berechnet, die zu einer Hemmung der Adhäsion um 50 % führt.

Es wurden die folgenden Testergebnisse erhalten:

| Verbindung des Beispiels | U937NCAM-1 Zelladhäsionstest IC₅₀ (µM) |
|---|---|
| 1 | 3,2 |
| 3 | 0,56 |
| 4 | 27,8 |
| 5 | 0,15 |
| 8 | 2,0 |
| 9 | 0,18 |
| 10 | 24,5 |
| 11 | 18,0 |
| 12 | 17,5 |
| 13 | 64,6 |
| 14 | 15,3 |
| 15 | 61,5 |
| 16 | 34,9 |
| 17 | 36,6 |

## Patentansprüche

1. Verbindung der Formel I 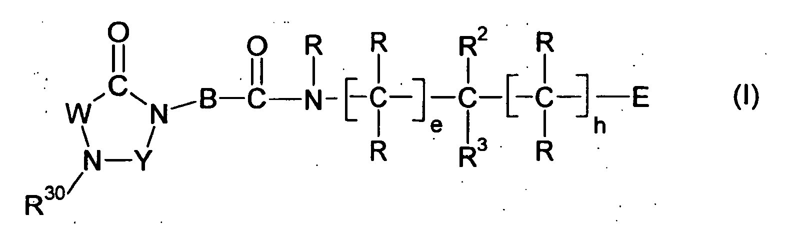 worin
W für den zweiwertigen Rest R¹-A-C(R¹³) steht;
Y für eine Carbonylgruppe steht;
B für einen zweiwertigen Methylenrest oder Ethylenrest steht, wobei der Methylenrest und der Ethylenrest unsubstituiert sind oder substituiert sind durch einen oder zwei gleiche oder verschiedene (C₁-C₈)-Alkylreste;
E für R¹⁰CO steht;
R für Wasserstoff oder (C₁-C₈)-Alkyl steht, wobei alle Reste R unabhängig voneinander die angegebenen Bedeutungen haben können und gleich oder verschieden sein können;
R¹-A- für (C₁-C₄)-Alkyl steht;
R² für Wasserstoff oder (C₁-C₈)-Alkyl steht;
R³ für Wasserstoff, (C₁-C₈)-Alkyl oder gegebenenfalls substituiertes (C₆-C₁₀)-Aryl steht;
R¹⁰ für Hydroxy oder (C₁-C₆)-Alkoxy steht;
R¹³ für (C₁-C₄)-Alkyl steht;
R³⁰ für einen der Reste R³²-CR=CR-R³¹- und R³²-C≡C=R³¹- steht, wobei die Reste R unabhängig voneinander die angegebenen Bedeutungen haben können und gleich oder verschieden sein können;
R³¹ für den zweiwertigen. Rest -R³³-R³⁴-R³⁵-R³⁶-steht, wobei R³⁶ an das Stickstoffatom im Imidazolidinring in der Formel I gebunden ist;
R³² für gegebenenfalls substituiertes (C₆-C₁₀)-Aryl oder im Arylrest gegebenenfalls substituiertes (C₆-C₁₀)-Aryl-(C₁-C₄)-alkyl steht;
R³³ für eine direkte Bindung oder (C₁-C₂)-Alkylen steht;
R³⁴ für einen zweiwertigen, gegebenenfalls substituierten (C₆-C₁₀)-Arylenrest steht;
R³⁵ für eine direkte Bindung oder (C₁-C₄)-Alkylen steht;
R³⁶ für eine direkte Bindung steht;
e und h unabhängig voneinander für 0 oder 1 stehen und gleich oder verschieden sein können;
in allen ihren stereoisomeren Formen und Mischungen davon in allen Verhältnissen, und ihre physiologisch verträglichen Salze.

2. Verbindung der Formel I gemäß Anspruch 1, worin B für unsubstituiertes Methylen steht oder für Methylen steht, das durch einen (C₁-C₈)-Alkylrest substituiert ist, in allen ihren stereoisomeren Formen und Mischungen davon in allen Verhältnissen, und ihre physiologisch,verträglichen Salze.

3. Verbindung der Formel Gemäß Anspruch 1 und/oder 2, worin R für Wasserstoff, Methyl oder Ethyl steht, in allen ihren stereoisomeren Formen und Mischungen davon in allen Verhältnisse, und ihre physiologisch verträglichen Salze.

4. Verbindung der Formel I gemäß einem oder mehreren der Ansprüche 1 bis 3, worin R² für Wasserstoff, Methyl oder Ethyl steht, in allen ihren stereoisomeren Formen und Mischungen davon in allen Verhältnissen, und ihre physiologisch verträglichen Salze.

5. Verbindung der Formel I gemäß einem oder mehreren der Ansprüche 1 bis 4, worin e für 0 steht und h für 1 steht, in allen, ihren stereoisomeren Formen und Mischungen davon in allen Verhältnissen, und ihre physiologisch verträglichen Salze.

6. Verfahren zur-Herstellung einer Verbindung der Formel I gemäß einem oder mehreren der Ansprüche 1 bis 5, **dadurch gekennzeichnet, daß** man eine Fragmentkondensation einer Verbindung der Formel II. 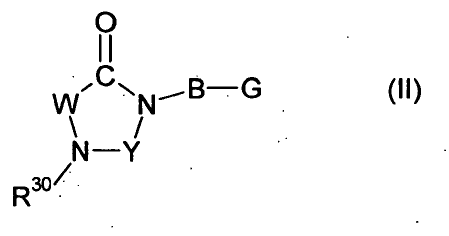 mit einer Verbindung der Formel III, 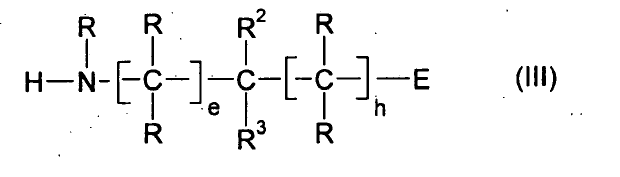 durchführt, wobei inden Formeln II und III die Gruppen W, Y, B, E, R, R², R³, R³⁰ sowie e und h wie in den Ansprüchen 1 bis 5 angegeben definiert sind oder auch funktionelle Gruppen in geschützter Form oder in Form von Vorstufen enthalten sein können, und wobei G für Hydroxycarbonyl, (C₁-C₆)-Alkoxycarbonyl oder aktivierte Carbonsäurederivate steht.

7. Verbindung der Formel. I gemäß einem oder mehreren der Ansprüche 1 bis 5 und/oder ihre physiologisch verträglichen Salze zur Verwendung als Arzneimittel.

8. Pharmazeutisches Präparat, **dadurch gekennzeichnet, daß** es eine oder mehrere Verbindungen der Formel I gemäß einem oder mehreren der Ansprüche 1 bis 5 und/oder ihre physiologisch verträglichen Salze und einen pharmazeutisch einwandfreien Träger enthält.

9. Verbindung der Formel I gemäß einem oder mehreren der Ansprüche 1 bis 5 und/oder ihre physiologisch verträglichen Salze zur Verwendung als Entzündungshemmstoffe.

10. Verbindung der Formel I gemäß einem oder mehreren der Ansprüche 1 bis 5 und/oder ihre physiologisch verträglichen Salze zur Verwendung in der Therapie oder Prophylaxe der Arthritis, der rheumatoiden Arthritis, der Polyarthritis, der inflammatory bowel disease, des systemischen Lupus erythematosus, der Multiplen Sklerose oder von inflammatorischen Erkrankungen des zentralen Nervensystems.

11. Verbindung der Formel I gemäß einem oder mehreren der Ansprüche 1 bis 5 und/oder ihre physiologisch verträgliche Salze zur Verwendung in der Therapie oder Prophylaxe von Asthma oder Allergien.

12. Verbindung der Formel I gemäß einem oder mehreren der Ansprüche 1 bis 5 und/oder ihre physiologisch verträgliche Salze zur Verwendung in der Therapie oder Prophylaxe von cardiovaskulären Erkrankungen, der Arteriosklerose, von Restenosen, von Diabetes, der Schädigung von Organtransplantaten, von Immunerkrankungen, von Autoimmunerkrankungen, von Tumorwachstum oder Tumormetastasierung oder der Malaria.

13. Verbindung der Formel Gemäß einem oder mehreren der Ansprüche 1 bis 5 und/oder ihre physiologisch verträglichen Salze zur in vitro Hemmung der Adhäsion und/oder der Migration von Leukozyten oder zur in vitro Hemmung des VLA-4-Rezeptors.

## Claims

1. A compound of the formula I, 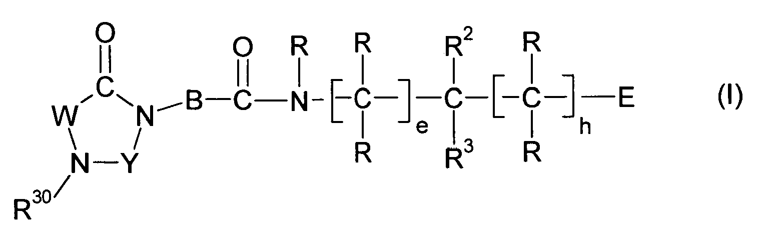 in which
W is the divalent radical R¹-A-C(R¹³);
Y is a carbonyl group;
B is a divalent methylene radical or ethylene radical, where the methylene radical and the ethylene radical are unsubstituted or are substituted by one or two identical or different (C₁-C₈)-alkyl radicals;
E is R¹⁰CO;
R is hydrogen or (C₁-C₈)-alkyl, where all radicals R independently of one another can have the meanings indicated and can be identical or different;
R¹-A- is (C₁-C₄)-alkyl;
R² is hydrogen or (C₁-C₈)-alkyl;
R³ is hydrogen, (C₁-C₈)-alkyl or optionally substituted (C₆-C₁₀)-aryl;
R¹⁰ is hydroxyl or (C₁-C₆)-alkoxy;
R¹³ is (C₁-C₄)-alkyl;
R³⁰ is one of the radicals R³²-CR=CR-R³¹- and R³²-C≡C-R³¹-, where the radicals R independently of one another can have the meanings indicated and can be identical or different;
R³¹ is the divalent radical -R³³-R³⁴-R³⁵-R³⁶-, where R³⁶ is bonded to the nitrogen atom in the imidazolidine ring in the formula I;
R³² is optionally substituted (C₆-C₁₀)-aryl or (C₆-C₁₀)-aryl-(C₁-C₄)-alkyl optionally substituted in the aryl radical;
R³³ is a direct bond or (C₁-C₂)-alkylene;
R³⁴ is a divalent, optionally substituted (C₆-C₁₀)-arylene radical;
R³⁵ is a direct bond or (C₁-C₄)-alkylene;
R³⁶ is a direct bond;
e and h independently of one another are 0 or 1 and can be identical or different; in all its stereoisomeric forms and mixtures thereof in all ratios, and its physiologically tolerable salts.

2. A compound of the formula I as claimed in claim 1, in which B is unsubstituted methylene or methylene which is substituted by a (C₁-C₈)-alkyl radical, in all its stereoisomeric forms and mixtures thereof in all ratios, and its physiologically tolerable salts.

3. A compound of the formula I as claimed in claim 1 and/or 2, in which R is hydrogen, methyl or ethyl, in all its stereoisomeric forms and mixtures thereof in all ratios, and its physiologically tolerable salts.

4. A compound of the formula I as claimed in one or more of claims 1 to 3, in which R² is hydrogen, methyl or ethyl, in all its stereoisomeric forms and mixtures thereof in all ratios, and its physiologically tolerable salts.

5. A compound of the formula I as claimed in one or more of claims 1 to 4, in which e is 0 and h is 1, in all its stereoisomeric forms and mixtures thereof in all ratios, and its physiologically tolerable salts.

6. A process for the preparation of a compound of the formula I as claimed in one or more of claims 1 to 5, which comprises carrying out a fragment condensation of a compound of the formula II 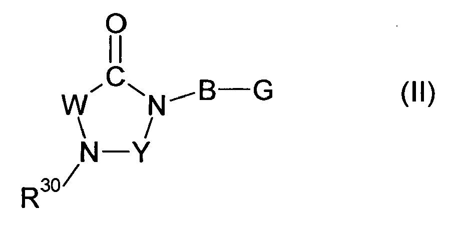 with a compound of the formula III, 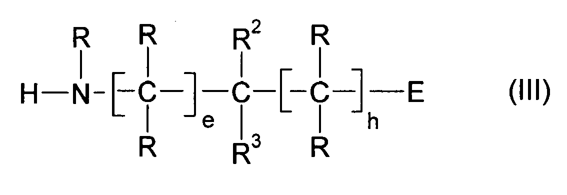 where, in the formulae II and III, the groups W, Y, B, E, R, R², R³, R³⁰ as well as e and h are as defined in claims 1 to 5 or alternatively functional groups can be contained in protected form or in the form of precursors, and where G is hydroxycarbonyl, (C₁-C₆)-alkoxycarbonyl or activated carboxylic acid derivatives.

7. A compound of the formula I as claimed in one or more of claims 1 to 5 and/or its physiologically tolerable salts for use as a medicament.

8. A pharmaceutical preparation which comprises one or more compounds of the formula I as claimed in one or more of claims 1 to 5 and/or its physiologically tolerable salts and a pharmaceutically innocuous vehicle.

9. A compound of the formula I as claimed in one or more of claims 1 to 5 and/or its physiologically tolerable salts for use as anti-inflammatories.

10. A compound of the formula I as claimed in one or more of claims 1 to 5 and/or its physiologically tolerable salts for use in the therapy or prophylaxis of arthritis, of rheumatoid arthritis, of polyarthritis, of inflammatory bowel disease, of systemic lupus erythematosus, of multiple sclerosis or of inflammatory disorders of the central nervous system.

11. A compound of the formula I as claimed in one or more of claims 1 to 5 and/or its physiologically tolerable salts for use in the therapy or prophylaxis of asthma or allergies.

12. A compound of the formula I as claimed in one or more of claims 1 to 5 and/or its physiologically tolerable salts for use in the therapy or prophylaxis of cardiovascular disorders, of arteriosclerosis, of restenoses, of diabetes, of damage to organ transplants, of immune disorders, of autoimmune disorders, of tumor growth or formation of tumor metastases or of malaria.

13. A compound of the formula I as claimed in one or more of claims 1 to 5 and/or its physiologically tolerable salts for the in vitro inhibition of the adhesion and/or the migration of leukocytes or for the in vitro inhibition of the VLA-4 receptor.

## Revendications

1. Composé de formule I 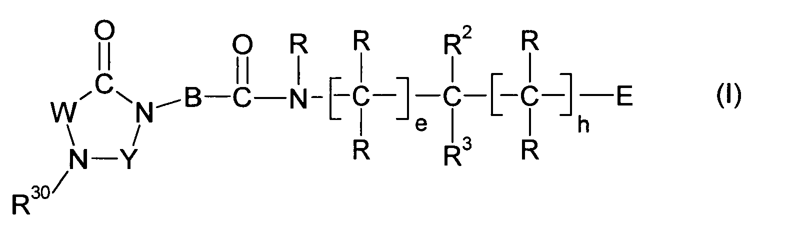 où
W représente le radical divalent R¹-A-C(R¹³) ;
Y représente un groupe carbonyle ;
B représente un radical méthylène ou éthylène divalent, où le radical méthylène et le radical éthylène sont non substitués ou substitués par un ou deux radicaux (C₁-C₈)-alkyle identiques ou différents ;
E représente R¹⁰CO ;
R représente hydrogène ou (C₁-C₈)-alkyle, où tous les radicaux R peuvent présenter, indépendamment l'un de l'autre, les significations indiquées et peuvent être identiques ou différents ;
R¹-A- représente (C₁-C₄)-alkyle ;
R² représente hydrogène ou (C₁-C₈)-alkyle ;
R³ représente hydrogène, (C₁-C₈)-alkyle ou (C₆-C₁₀)-aryle éventuellement substitué ;
R¹⁰ représente hydroxy ou (C₁-C₆)-alcoxy ;
R¹³ représente (C₁-C₄)-alkyle ;
R³⁰ représente un des radicaux R³²-CR=CR-R³¹- et R³²-C≡C-R³¹-, où les radicaux R peuvent présenter, indépendamment l'un de l'autre, les significations indiquées et peuvent être identiques ou différents ;
R³¹ représente le radical divalent -R³³-R³⁴-R³⁵-R³⁶-, où R³⁶ est lié à l'atome d'azote dans le cycle imidazolidine dans la formule 1 ;
R³² représente (C₆-C₁₀)-aryle éventuellement substitué ou (C₆-C₁₀)-aryl-(C₁-C₄)-alkyle éventuellement substitué dans le radical aryle ;
R³³ représente une liaison directe ou (C₁-C₂)-alkylène ;
R³⁴ représente un radical (C₆-C₁₀)-arylène divalent, éventuellement substitué ;
R³⁵ représente une liaison directe ou (C₁-C₄)-alkylène ;
R³⁶ représente une liaison directe ;
e et h valent, indépendamment l'un de l'autre, 0 ou 1 et peuvent être identiques ou différents ;
dans toutes ses formes stéréo-isomères et leurs mélanges, dans tous les rapports, et ses sels physiologiquement acceptables.

2. Composé de formule I selon la revendication 1, où B représente méthylène non substitué ou représente méthylène qui est substitué par un radical (C₁-C₈)-alkyle, dans toutes ses formes stéréo-isomères et leurs mélanges, dans tous les rapports, et ses sels physiologiquement acceptables.

3. Composé de formule I selon la revendication 1 et/ou 2, où R représente hydrogène, méthyle ou éthyle, dans toutes ses formes stéréo-isomères et leurs mélanges, dans tous les rapports, et ses sels physiologiquement acceptables.

4. Composé de formule I selon l'une ou plusieurs des revendications 1 à 3, où R² représente hydrogène, méthyle ou éthyle, dans toutes ses formes stéréo-isomères et leurs mélanges, dans tous les rapports, et ses sels physiologiquement acceptables.

5. Composé de formule I selon l'une ou plusieurs des revendications 1 à 4, où e vaut 0 et h vaut 1, dans toutes ses formes stéréo-isomères et leurs mélanges, dans tous les rapports, et ses sels physiologiquement acceptables.

6. Procédé pour la préparation d'un composé de formule I selon l'une ou plusieurs des revendications 1 à 5, **caractérisé en ce qu'**on réalise une condensation fragmentaire d'un composé de formule II 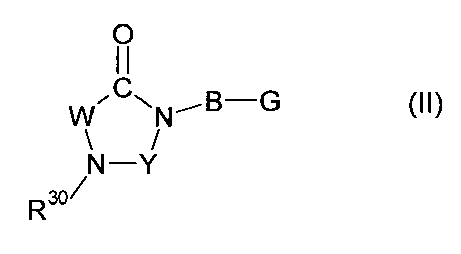 avec un composé de formule III 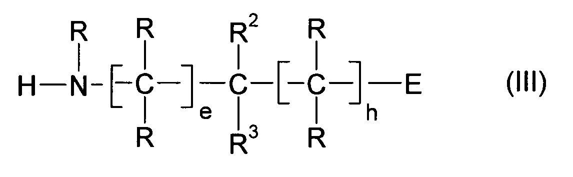 où, dans les formules II et III, les groupes W, Y, B, E, R, R², R³ et R³⁰ ainsi que e et h sont définis comme indiqué dans les revendications 1 à 5 ou des groupes fonctionnels peuvent également être contenus sous forme protégée ou sous forme de précurseurs et où G représente hydroxycarbonyle, (C₁-C₆)-alcoxycarbonyle ou des dérivés activés d'acide carboxylique.

7. Composé de formule 1 selon l'une ou plusieurs des revendications 1 à 5 et/ou ses sels physiologiquement acceptables destiné à une utilisation comme médicament.

8. Préparation pharmaceutique, **caractérisée en ce qu'**elle contient un ou plusieurs composés de formule 1 selon l'une ou plusieurs des revendications 1 à 5 et/ou ses/leurs sels physiologiquement acceptables et un support pharmaceutiquement irréprochable.

9. Composé de formule 1 selon l'une ou plusieurs des revendications 1 à 5 et/ou ses sels physiologiquement acceptables destinés à une utilisation comme anti-inflammatoires.

10. Composé de formule 1 selon l'une ou plusieurs des revendications 1 à 5 et/ou ses sels physiologiquement acceptables destinés à une utilisation dans la thérapie ou la prophylaxie de l'arthrite, de l'arthrite rhumatoïde, de la polyarthrite, de maladie intestinale inflammatoire, du lupus érythémateux systémique, de la sclérose en plaques ou de maladies inflammatoires du système nerveux central.

11. Composé de formule I selon l'une ou plusieurs des revendications 1 à 5 et/ou ses sels physiologiquement acceptables destinés à une utilisation dans la thérapie ou la prophylaxie de l'asthme ou d'allergies.

12. Composé de formule 1 selon l'une ou plusieurs des revendications 1 à 5 et/ou ses sels physiologiquement acceptables destinés à une utilisation dans la thérapie ou la prophylaxie de maladies cardiovasculaires, de l'artériosclérose, de resténoses, du diabète, de la dégradation des transplants d'organes, de maladies immunitaires, de maladies auto-immunitaires, de la croissance de tumeurs ou de la formation de métastases de tumeurs ou de la malaria.

13. Composé de formule 1 selon l'une ou plusieurs des revendications 1 à 5 et/ou ses sels physiologiquement acceptables destinés à l'inhibition in vitro de l'adhérence et/ou de la migration de leucocytes ou à l'inhibition in vitro du récepteur de VLA-4.
